(19) Europäisches Patentamt
European Patent Office
Office européen des brevets

(11) EP 3 199 589 B1

(12) **EUROPÄISCHE PATENTSCHRIFT**

(45) Veröffentlichungstag und Bekanntmachung des Hinweises auf die Patenterteilung:
**26.12.2018 Patentblatt 2018/52**

(51) Int Cl.:
***C08L 67/08*** (2006.01) ***A61Q 19/10*** (2006.01)
***A61K 8/86*** (2006.01) ***A61K 8/39*** (2006.01)

(21) Anmeldenummer: **17150500.1**

(22) Anmeldetag: **06.01.2017**

(54) **POLYGLYCERINALKOXYLATESTER, DEREN HERSTELLUNG UND VERWENDUNG**
POLYGLYCERIN ALKOXYLATE ESTERS, THEIR PREPARATION AND USE
ESTER D'ALCOXYLATE POLY GLYCÉROL SA FABRICATION ET SON UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.01.2016 EP 16153288**

(43) Veröffentlichungstag der Anmeldung:
**02.08.2017 Patentblatt 2017/31**

(73) Patentinhaber: **Evonik Degussa GmbH**
**45128 Essen (DE)**

(72) Erfinder:
- **Brandt, Kathrin Daniela**
  **40476 Düsseldorf (DE)**
- **Lobert, Matthias**
  **45309 Essen (DE)**
- **Schuch, Dominik**
  **40221 Düsseldorf (DE)**
- **Venzmer, Joachim**
  **45239 Essen (DE)**
- **Schwab, Peter**
  **45133 Essen (DE)**
- **Bednorz, Beata**
  **45219 Essen (DE)**
- **Brösgen, Anja**
  **45138 Essen (DE)**
- **Blaschek, Svenja**
  **45899 Gelsenkirchen (DE)**

(56) Entgegenhaltungen:
**EP-A2- 0 264 826**
**EP-A2- 0 919 219**
**EP-A2- 1 055 407**
**EP-A2- 1 344 518**
**EP-A2- 1 518 900**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 199 589 B1

## Beschreibung

### Gebiet der Erfindung

Gegenstand der Erfindung sind Polyglycerinalkoxylatester sowie deren Herstellung und Verwendung.

### Stand der Technik

Die Stoffklasse der Glycerin- und Polyglycerinalkoxylatester ist bekannt und es sind verschiedene Anwendungen für diese Stoffklasse beschrieben. Dazu zählen die Verwendung als Antischaummittel, wie in der EP0809527 und EP0264826 offenbart, als Emulgatoren in der Emulsionspolymerisation, wie in der DE10124547 offenbart, als Inhaltsstoff von Pestizidzubereitungen, wie in der WO0108481 offenbart, sowie der Einsatz im Bereich Kosmetik als Verdicker, Solubilisatoren, Konditionier- und Rückfettungsmittel, wie beispielsweise in DE3239564, DE19753108 und DE2024051 offenbart. Die entsprechenden Glycerinesterethoxylate werden im Markt standardmäßig als Rückfettungsmittel (z.B. PEG-7 Glyceryl Cocoate = TEGOSOFT GC) und Verdicker (z.B. PEG-200 Hydrogenated Glyceryl Palmate = REWODERM LI S 80) in wässrigen Tensidformulierungen eingesetzt.
Im Stand der Technik sind auch Polyglycerinalkoxylatester als Verdicker von kosmetischen Rinse-Off-Formulierungen beschrieben: So offenbart die EP1344518 kosmetische und pharmazeutische Zubereitungen enthaltend einen oxalkylierten Polyglycerinester mit einem Polymerisationsgrad des Polyglycerins von 1 bis 30, mit 50 bis 250 Alkoxyeinheiten pro Molekül, und die EP1518900 Zusammensetzungen, enthaltend a) einen oder mehrere oxalkylierte Polyglycerinester mit einem Polymerisationsgrad des Polyglycerins von 1,5 bis 10, mit 50 bis 250 Alkoxyeinheiten pro Molekül, b) ein organisches Lösungsmittel oder organisches Lösungsmittelgemisch, und c) Wasser.

Trotz einer breiten verfügbaren Palette an Verdickern für wässrige Tensidsysteme besteht marktseitig weiterhin großes Interesse an neuen noch effizienteren Verdickern. Gerade die Bereitstellung einfach dosierbarer und hocheffizienter Verdicker bietet dem Anwender erhebliche Vorteile. Gerade letzterer Punkt würde dem Anwender ermöglichen mit geringeren Wirkstoffmengen den gleichen verdickenden Effekt zu erzeugen und somit Ressourcen schonen. Aufgabe der vorliegenden Patentschrift war also somit die Bereitstellung neuer, hocheffizienter, leicht dosierbarer Verdicker.

### Beschreibung der Erfindung

Überraschenderweise wurde gefunden, dass Polyglycerinalkoxylatester mit mehr als 250 Alkoxyeinheiten und mit drei und mehr Acylresten pro Molekül als signifikant effektivere Verdicker von wässrigen Tensidsystemen fungieren als die im Stand der Technik beschriebenen Strukturen.

Gegenstand der vorliegenden Erfindung sind daher Polyglycerinalkoxylatester mit mehr als 250 Alkoxyeinheiten und mit drei und mehr Acylresten pro Molekül sowie deren Herstellung und Verwendung.

Ein Vorteil der vorliegenden Erfindung ist die verbesserte Verdickungsleistung der erfindungsgemäßen Polyglycerinalkoxylatester gegenüber bereits bekannten und marktüblichen Ethoxylatestern in wässrigen tensidischen Formulierungen. Demgemäß können in Gegenwart der erfindungsgemäßen Polyglycerinalkoxylatester höhere Viskositäten in wässrigen tensidischen Formulierungen mit analoger Zusammensetzung erreicht werden als mit nicht erfindungsgemäßen Verdickern. Bei Austausch eines marktüblichen Verdickers durch einen erfindungsgemäßen Polyglycerinalkoxylatester wird daher bei gleichbleibender Tensidkonzentration eine geringere Menge des erfindungsgemäßen Polyglycerinalkoxylatesters benötigt, um eine unveränderte Viskosität zu erhalten. Alternativ kann beim Austausch der gleichen Menge nicht erfindungsgemäßer Ethoxylatester durch die erfindungsgemäßen Polyglycerinalkoxylatester auch die Menge der eingesetzten Tenside oder die Menge des Kochsalzes in der Formulierung ohne Viskositätsverlust reduziert werden. Demgemäß ermöglichen die erfindungsgemäßen Polyglycerinalkoxylatester also generell eine Reduzierung der Gesamtkonzentration einer wässrigen tensidischen Formulierung, wodurch sich eine gesteigerte Effizienz ergibt. Darüber hinaus wirken sich reduzierte Primärtensid- und Salzgehalte vorteilhaft auf die Mildheit, das Hautgefühl und die Korrosivität der jeweiligen tensidischen Formulierungen aus.
Ein weiterer Vorteil der hier beschriebenen Polyglycerinalkoxylatester ist, dass diese ein angenehmes Hautgefühl in kosmetischen Formulierungen erzeugen können.
Ein weiterer Vorteil ist, dass die hier beschriebenen Polyglycerinalkoxylatester die Reizwirkung von tensidischen Formulierungen abmildern können.
Ein weiterer Vorteil der hier beschriebenen Polyglycerinalkoxylatester ist, dass diese ohne Zufuhr von Wärmeenergie bei 20 bis 25 °C in wässrige tensidische Formulierungen eingearbeitet werden können.
Ein weiterer Vorteil ist, dass die hier beschriebenen Polyglycerinalkoxylatester eine hohe Wasserlöslichkeit besitzen und damit die Herstellung glasklarer tensidischer Formulierungen ermöglichen.
Ein weiterer Vorteil der erfindungsgemäßen Produkte ist, dass diese in Wasser unter Rühren eine sehr geringe Schaumbildung zeigen.

Noch ein Vorteil ist, dass die hier beschriebenen Polyglycerinalkoxylatester die Anschäumbarkeit und Schaummenge tensidischer Formulierungen nicht beeinflussen, aber die Cremigkeit des Schaums verbessern können.
Ein weiterer Vorteil der erfindungsgemäßen Produkte ist, dass diese eine gute Stabilität gegenüber Oxidation aufweisen und damit stabil bzgl. Farbe, Geruch und Aussehen sind.

Gegenstand der vorliegenden Erfindung sind daher Polyglycerinalkoxylatester der allgemeinen Formel (I)

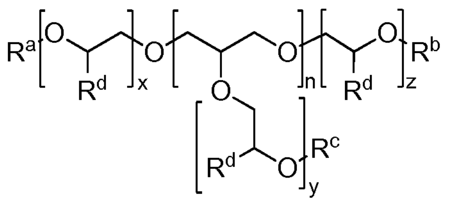

allgemeine Formel (I)

mit

$R^a$, $R^b$, $R^c$ = unabhängig voneinander, gleich oder verschieden, ausgewählt aus H oder Acylrest einer organischen Säure, bevorzugt H oder Acylrest einer Fettsäure, besonders bevorzugt H oder Acylrest einer Fettsäure mit 16 bis 22 Kohlenstoffatomen, mit der Maßgabe, dass pro Molekül im Mittel 3 bis 6, besonders bevorzugt 3,5 bis 5,5, insbesondere bevorzugt 4 bis 5 der Reste $R^a$, $R^b$, $R^c$ ungleich H sind,
$R^d$ = unabhängig voneinander, gleich oder verschieden, ausgewählt aus H, Alkyl- oder Aryl-, bevorzugt H, Methyl-, Ethyl-, besonders bevorzugt H oder Methyl, insbesondere H,
n = 1 bis 16, bevorzugt 2 bis 14, besonders bevorzugt 3 bis 11, ganz besonders bevorzugt 4 bis 9, x, y, z = unabhängig voneinander, gleich oder verschieden, 0 bis 200, bevorzugt 30 bis 100,
besonders bevorzugt 40 bis 80, mit der Maßgabe, dass die Gesamtsumme $x + n \cdot y + z$ pro Molekül im Mittel 251 bis 750, bevorzugt 300 bis 600, besonders bevorzugt 350 bis 550, beträgt.

Die erfindungsgemäßen Polyglycerinalkoxylatester stellen Mischungen von unterschiedlichen Substanzen dar; daher ist dem Fachmann klar, dass die angegeben Zahlenwerte Mittelwerte über die Mischung darstellen können.
Unter dem Begriff "Polyglycerin" im Sinne der vorliegenden Erfindung und wie in der allgemeinen Formel (I) dargestellt, ist ein Polyglycerin zu verstehen, welches auch Glycerin enthalten kann. Somit ist zur Berechnung von Mengen, Massen und dergleichen gegebenenfalls ein Glycerinanteil mit zu berücksichtigen. Das Polyglycerin stellt aufgrund seiner polymeren Eigenschaft eine statistische Mischung verschiedener Verbindungen dar. Polyglycerin kann ausgebildete Etherbindungen zwischen zwei primären, einer primären und einer sekundären sowie zwei sekundären Positionen der Glycerinmonomere aufweisen. Aus diesem Grund besteht das Polyglycerin-Grundgerüst üblicherweise nicht ausschließlich aus linear verknüpften Glycerin-Einheiten, sondern kann auch Verzweigungen und Cyclen enthalten. Für Details siehe z.B. "Original synthesis of linear, branched and cyclic oligoglycerol standards", Cassel et al., J. Org. Chem. 2001, 875-896. Entsprechende Strukturen sind von der in dieser Hinsicht vereinfachten, allgemeinen Formel (I) miterfasst.
Es ist offenbar, dass die n-fach enthaltenen Reste $R^c$ gleich sein oder sich untereinander unterscheiden können. Ebenso kann der n-fach enthaltene Index y in den n Einheiten gleich oder verschieden sein.
Zur Bestimmung von Parametern oder Messwerten werden vorzugsweise die nachfolgend beschriebenen Methoden verwendet. Insbesondere werden diese Methoden in den Beispielen des vorliegenden Schutzrechts verwendet.
Gewichtsmittlere und zahlenmittlere Molekulargewichte werden im Rahmen dieser Erfindung für die hergestellten Polyglycerinalkoxylate und Polyglycerinalkoxylatester kalibriert gegen einen Polypropylenglykolstandard durch Gelpermeationschromatographie (GPC) bestimmt. Die GPC wurde durchgeführt auf einem Agilent 1100 ausgestattet mit einem RI-Detektor und einer SDV 1000/10000 Å Säulenkombination bestehend aus einer 0,8 cm x 5 cm Vorsäule und zwei 0,8 cm x 30 cm Hauptsäulen bei einer Temperatur von 30°C und einer Fließrate von 1 mL/min (mobile Phase: THF). Die Probenkonzentration betrug 10 g/L und das Injektionsvolumen 20 μL. Die Polydispersität entspricht dem Quotienten $M_w$ durch $M_n$ (PDI = $M_w/M_n$).

Zur Bestimmung des Alkoxylierungsgrades wird die $^1$H-NMR-Spektroskopie genutzt. Die NMR-Spektren werden mit einem 400 MHz Spektrometer der Firma Bruker unter Einsatz eines QMP-Kopfes gemessen. Die zu untersuchende Probe wird in einem geeigneten deuterierten Lösungsmittel (z.B. Methanol) gelöst und in 5 mm oder ggf. 10 mm NMR-Röhrchen überführt.
Die charakteristischen Protonensignale eines beispielhaften, erfindungsgemäßen Polyglycerinalkoxylatesters sind in der folgenden schematischen Abbildung gezeigt, die einer vereinfachten Darstellung von Formel (I) entspricht, bei der, aus Gründen der Übersichtlichkeit exklusiv Stearinsäure als Fettsäurefragment und Ethylenoxid, im Folgenden auch als EO abgekürzt, als Alkylenoxidmonomer gezeigt sind. Sinngemäß lassen sich die folgenden Erläuterungen auch auf Strukturen übertragen, die andere Fettsäuren oder Alkylenoxide als Monomerbausteine enthalten.

Neben den einzelnen charakteristischen Fragmenten (von links nach rechts gezeigt) Fettsäure (Stearinsäure), Ethylenoxid und (Poly)Glycerinfragment sind noch die charakteristischen Protonen mit den Indizes $H^a$ bis $H^j$ genannt, sowie deren Verschiebungen im $^1$H-NMR-Spektrum. Das NMR wurde kalibriert auf das Signal des Lösungsmittels, im konkreten Fall auf das Signal des Methanols bei 3,3 ppm.

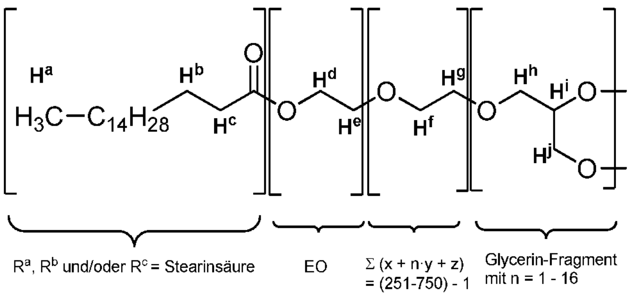

$R^a$, $R^b$ und/oder $R^c$ = Stearinsäure

EO

$\Sigma$ (x + n·y + z) = (251-750) - 1

Glycerin-Fragment mit n = 1 - 16

| | |
|---|---|
| $\delta$ ($H^a$) | ~ 0,9 ppm |
| $\delta$ ($H^b$) | ~ 1,6 ppm |
| $\delta$ ($H^c$) | ~ 2,3 ppm |
| $\delta$ ($H^d$) | ~ 4,2 ppm |
| $\delta$ ($H^e$ - $H^j$) | ~ 3,4 - 3,9 ppm |

Eine quantitative Veresterung der Fettsäuren kann aus den Integralverhältnissen der Signale der Gruppen $H^a$, $H^b$ und/oder $H^c$ der Acylreste zum Signal der Gruppe $H^d$ der acylierten EthylenoxidEinheiten der Polyalkylenoxidketten abgeleitet werden. Alle Signale weisen im Rahmen der Messgenauigkeit der Methode (Integrationsfehler ca. 5%) die gleichen Integralwerte pro Wasserstoff-Atom der jeweiligen Gruppe auf.

Der Alkoxylierungsgrad kann aus den Integralverhältnissen der Signalgruppe $H^e$ bis $H^j$ beispielsweise zu den Signalen der Gruppe $H^a$ oder $H^c$ berechnet werden. Bevorzugt wird für die Berechnung des Alkoxylierungsgrades das Signal $H^a$ als repräsentatives Signal für den Fettsäurerest herangezogen.

Bei der mathematischen Berechnung der Protonenzahl der Alkylenoxid-Einheiten der Signalgruppe $H^e$ bis $H^j$ sind jedoch die (Poly)Glycerinprotonen in der Bilanzierung zu berücksichtigen und ebenso, dass die Protonen $H^d$, die ja auch vom Alkylenoxid stammen, ein separates Signal bedingen.

Liegt also beispielsweise ein Tetrastearat von Polyglycerin-5 mit 400 mol EO vor, so kann die Bilanzierung folgendermaßen erfolgen: Das Integral des Signales $H^a$ muss durch 12 (4 Moläquivalente Fettsäure mit je einer $CH_3$-Gruppe) dividiert werden, um den Anteil, welcher einem Proton entspricht, zu ermitteln. Wenn man nun das Integral der Signalgruppe $H^e$ bis $H^j$ durch den zuvor ermittelten Integralanteil, welcher einem Proton entspricht, dividiert, so ergibt sich die Protonenanzahl dieser Signalgruppe. Von diesem Wert müssen nun noch die Anzahl der Protonen des (Poly)glycerins subtrahiert werden (also minus 25, da 5 mol Glycerin mit je 5 Wasserstoffen enthalten sind) und die verbleibenden Protonen ergeben unter Berücksichtigung des Integrals des Signals $H^d$, die Gesamtheit aller Ethylenoxid-Signale. Wenn man diese Anzahl durch vier (1 mol EO enthält 4 Protonen) dividiert, erhält man schließlich im konkreten Fall den Ethoxylierungsgrad.

Nasschemische Analysen werden gemäß internationaler Standardmethoden durchgeführt (Säurezahl: DGF C-V 2, Ph.Eur. 2.5.1; Hydroxylzahl: DGF C-V 17 a (53), Ph. Eur. 2.5.3 method A; Verseifungszahl: DGF C-V 3, Ph.Eur. 2.5.6).

Der Polymerisationsgrad n lässt sich über die Hydroxylzahl des Polyglycerins ermitteln, wobei der mittlere Polymerisationsgrad n mit der Hydroxylzahl des zugrundeliegenden Polyglycerins über folgende Gleichung verknüpft ist:

$$n = \frac{\frac{2000 \bullet M(KOH)}{OHZ} - M(Wasser)}{\left[ [M(Glycerin) - M(Wasser)] - \frac{1000 \bullet M(KOH)}{OHZ} \right]} \qquad \text{Formel (II)}$$

mit M = molare Masse; OHZ = Hydroxylzahl des freien Polyglycerins.

Alle angegebenen Prozent (%) sind, wenn nicht anders angegeben, Massenprozent.

Bevorzugte Polyglycerinalkoxylatester der vorliegenden Erfindung weisen Reste $R^a$, $R^b$, $R^c$ auf, bei denen mindestens 40 % der Acylreste 18 Kohlenstoffatome enthalten, wobei es sich bei diesen ganz besonders bevorzugt um Stearinsäurereste handelt, wobei die Prozente sich auf die numerische Anzahl aller im Polyglycerinalkoxylatester enthaltenen Reste $R^a$, $R^b$, $R^c$ beziehen.

Bevorzugte Polyglycerinalkoxylatester der vorliegenden Erfindung sind dadurch gekennzeichnet, dass

$R^a$, $R^b$, $R^c$ = H oder Acylrest einer Fettsäure mit 16 bis 22 Kohlenstoffatomen,

mit der Maßgabe, dass pro Molekül im Mittel 3,5 bis 6 der Reste $R^a$, $R^b$, $R^c$ ungleich H sind,

$R^d$ = H,

n = 3 bis 11,
x, y, z = unabhängig voneinander, gleich oder verschieden, 30 bis 200,
mit der Maßgabe, dass die Gesamtsumme x + n·y + z pro Molekül im Mittel 300 bis 750 beträgt.

Bevorzugte Polyglycerinalkoxylatester der vorliegenden Erfindung sind dadurch gekennzeichnet, dass
$R^a$, $R^b$, $R^c$ = H oder Acylrest einer Fettsäure mit 16 bis 22 Kohlenstoffatomen,
mit der Maßgabe, dass pro Molekül im Mittel 4 bis 5 der Reste $R^a$, $R^b$, $R^c$ ungleich H,
$R^d$ = H,
n = 4 bis 9,
x, y, z = 40 bis 80,
mit der Maßgabe, dass die Gesamtsumme x + n·y + z pro Molekül im Mittel 350 bis 550, beträgt.
In diesem Zusammenhang ist es insbesondere bevorzugt, dass die Polyglycerinalkoxylatester der vorliegenden Erfindung Reste $R^a$, $R^b$, $R^c$ aufweisen, bei denen mindestens 40 % der Acylreste der Fettsäure Stearinsäurereste sind, wobei die Prozente sich auf die numerische Anzahl aller im Polyglycerinalkoxylatester enthaltenen Reste $R^a$, $R^b$, $R^c$ beziehen.

Prinzipiell können die erfindungsgemäßen Polyglycerinalkoxylatester nach beliebigen Verfahren, die dem Stand der Technik, wie beispielsweise EP1344518 und EP1518900, entnommen werden können, hergestellt werden.

Bevorzugt werden die erfindungsgemäßen Polyglycerinalkoxylatester nach dem im Folgenden beschriebenen Verfahren zur Herstellung von Polyglycerinalkoxylatestern hergestellt, welches ebenfalls ein Gegenstand der vorliegenden Erfindung ist.

Das erfindungsgemäße Verfahren zur Herstellung von Polyglycerinalkoxylatestern umfasst die Verfahrensschritte

A) Bereitstellen eine Polyglycerins mit einem Polymerisationsgrad von 1,5 bis 16, bevorzugt 2 bis 14, besonders bevorzugt 3 bis 11, ganz besonders bevorzugt 4 bis 9,
B) Alkoxylierung mit 251 bis 750, bevorzugt 300 bis 600, besonders bevorzugt 350 bis 550 Mol Alkylenoxid ausgewählt aus der Gruppe Ethylenoxid, Propylenoxid, Butylenoxid und Dodecenoxid, insbesondere Ethylenoxid, pro im gesamten Verfahren eingesetzten Mol Polyglycerin, und
C) Veresterung mit 3 bis 6, besonders bevorzugt 3,5 bis 5,5, insbesondere bevorzugt 4 bis 5, Mol mindestens einer ausgewählt aus organischen Säuren, bevorzugt Fettsäuren, insbesondere Fettsäuren mit 16 bis 22 Kohlenstoffatomen, pro im gesamten Verfahren eingesetzten Mol Polyglycerin.

Das erfindungsgemäße Verfahren zur Herstellung von Polyglycerinalkoxylatestern kann mindestens einen weiteren Verfahrensschritt wie beispielsweise

D) Aufreinigung des Polyglycerinalkoxylatesters
umfassen.

Das Polyglycerin für Verfahrensschritt A) kann bereitgestellt werden durch verschiedene konventionelle Methoden wie beispielsweise Polymerisation von Glycidol (z.B. basenkatalysiert), Polymerisation von Epichlorhydrin (beispielsweise in Gegenwart äquimolarer Mengen einer Base wie NaOH) oder Polykondensation von Glycerin. Letzteres erfolgt beispielsweise in Gegenwart katalytischer Mengen einer Base wie beispielsweise NaOH oder KOH. Geeignete Reaktionsbedingungen sind Temperaturen zwischen 220 und 260 °C sowie ein reduzierter Druck in einem Bereich zwischen 20 bis 800 mbar, insbesondere zwischen 50 und 500 mbar, wodurch eine erleichterte Wasserentfernung möglich ist. Entsprechende Verfahren lassen sich Standardlehrwerken der Chemie wie beispielsweise dem Römpp online, Thieme Verlag, mit Stand vom 31.12.2015, entnehmen.
Verfahrensschritt B), eine Alkoxylierung, ist eine bekannte Synthese der organischen Chemie mit hinlänglich bekannten Prozessparametern und wird zum Beispiel in den Kapiteln "Surfactants" und/oder "Polyoxyalkylenes" in Ullmann's Encyclopedia of Industrial Chemistry, sowie in "Alkylene_Oxides_and_Their_Polymers" von F. E. Bailey und J. V. Koleske, Marcel Dekker Inc. 1991 beschrieben.
Verfahrensschritt C), eine Veresterung, ist eine bekannte Synthese der organischen Chemie mit hinlänglich bekannten Prozessparametern und wird in Standardlehrwerken wie beispielsweise dem Organikum, Wiley-VCH Verlag, beschrieben. Polyglycerinestersynthesen im Speziellen sind beschrieben in beispielsweise Fette, Seifen, Anstrichm. 82, 93 (1980) oder Tenside, Deterg. 23, 320 (1986).

Die Verfahrensschritte können im erfindungsgemäßen Verfahren in der Reihenfolge "A), B), C)", "A), C), B)" durchgeführt werden, wobei "A), C), B)" erfindungsgemäß bevorzugt ist, da die auf diese Weise erhältlichen Polyglycerinalkoxylatester eine erhöhte Lagerstabilität und eine weniger konzentrationsabhängige Verdickungsleistung in wässrigen tensidischen Formulierungen aufweisen.

Bevorzugte erfindungsgemäße Verfahren sind dadurch gekennzeichnet, dass in Verfahrensschritt A) ein Polyglycerin mit einem Polymerisationsgrad von 3 bis 11,

in Verfahrensschritt B) 300 bis 750 Mol Ethylenoxid, pro im gesamten Verfahren eingesetzten Mol Polyglycerin, und
in Verfahrensschritt C) 3,5 bis 6 Mol mindestens einer ausgewählt aus Fettsäuren mit 16 bis 22 Kohlenstoffatomen pro im gesamten Verfahren eingesetzten Mol Polyglycerin, eingesetzt werden.

Besonders bevorzugte erfindungsgemäße Verfahren sind dadurch gekennzeichnet, dass in Verfahrensschritt A) ein Polyglycerin mit einem Polymerisationsgrad von 4 bis 9,
in Verfahrensschritt B) 350 bis 550 Mol Ethylenoxid, pro im gesamten Verfahren eingesetzten Mol Polyglycerin, und
in Verfahrensschritt C) 4 bis 5 Mol mindestens einer ausgewählt aus Fettsäuren mit 16 bis 22 Kohlenstoffatomen pro im gesamten Verfahren eingesetzten Mol Polyglycerin, eingesetzt werden. In diesem Zusammenhang ist es insbesondere bevorzugt, dass Fettsäuren mit 16 bis 22 Kohlenstoffatomen eingesetzt werden, bei denen mindestens 40 Mol%, bezogen auf alle Fettsäuren mit 16 bis 22 Kohlenstoffatomen, Stearinsäure ist.

Die nach dem erfindungsgemäßen Verfahren erhältlichen Polyglycerinalkoxylatester weisen überraschende anwendungstechnische Eigenschaften auf und sind somit ebenfalls Gegenstand der vorliegenden Erfindung, wobei nach oben als bevorzugt ausgewiesenen, erfindungsgemäßen Verfahren erhältliche Polyglycerinalkoxylatester erfindungsgemäß natürlich entsprechend bevorzugt sind.

Die erfindungsgemäßen Polyglycerinalkoxylatester und die nach dem erfindungsgemäßen Verfahren erhältlichen Polyglycerinalkoxylatester lassen sich vorteilhaft in Formulierungen, insbesondere in den nachfolgend aufgeführten erfindungsgemäßen Formulierungen, verwenden, so zum Beispiel als Verdicker.

Ein weiterer Gegenstand sind somit Formulierungen enthaltend die erfindungsgemäßen Polyglycerinalkoxylatester und/oder die nach dem erfindungsgemäßen Verfahren erhältlichen Polyglycerinalkoxylatester, wobei es sich bei den erfindungsgemäßen Formulierungen bevorzugt um wässrige Formulierungen, insbesondere um wässrige tensidische Formulierungen handelt. Unter dem Begriff "wässrige Formulierung" im Zusammenhang mit der vorliegenden Erfindung ist eine Formulierung zu verstehen, die mindestens 5 Gew.-% Wasser, bezogen auf die betrachtete Gesamtzusammensetzung enthält.
In den erfindungsgemäßen Formulierungen sind bevorzugt außer den erfindungsgemäßen Polyglycerinalkoxylatestern und/oder den nach dem erfindungsgemäßen Verfahren erhältlichen Polyglycerinalkoxylatestern keine weiteren Polyglycerinalkoxylatester enthalten; enthält eine bevorzugte erfindungsgemäße Formulierung bevorzugte Polyglycerinalkoxylatester und/oder nach einem bevorzugten erfindungsgemäßen Verfahren erhältliche Polyglycerinalkoxylatester, so sind bevorzugt in der Formulierung auch nur diese Polyglycerinalkoxylatester enthalten.

Bevorzugte erfindungsgemäße Formulierungen sind dadurch gekennzeichnet, dass sie die erfindungsgemäßen Polyglycerinalkoxylatester und/oder die nach dem erfindungsgemäßen Verfahren erhältlichen Polyglycerinalkoxylatester in einer Menge von 0,01 Gew.-% bis 20 Gew.-%, bevorzugt 0,05 Gew.-% bis 10 Gew.-%, insbesondere 0,1 Gew.-% bis 5 Gew.-%, enthalten, wobei sich die Gewichtsprozente auf die Gesamtzusammensetzung beziehen.

Eine erfindungsgemäße wässrige tensidische Formulierung ist dadurch gekennzeichnet, dass sie zusätzlich mindestens ein Tensid, insbesondere in einer Gesamtmenge von insgesamt 0,1 Gew.-% bis 20 Gew.-%, bevorzugt 1,0 Gew.-% bis 15 Gew.-%, insbesondere 5,0 Gew.-% bis 10 Gew.-%, enthält, wobei sich die Gewichtsprozente auf die Gesamtzusammensetzung beziehen.
Unter dem Begriff "Tensid" im Zusammenhang mit der vorliegenden Erfindung werden organische Substanzen mit grenzflächenaktiven Eigenschaften verstanden, die die Fähigkeit besitzen, die Oberflächenspannung von Wasser bei 20°C und bei einer Konzentration von 0,5 Gew.-% bezogen auf die Gesamtzusammensetzung auf unter 45 mN/m zu verringern. Die Oberflächenspannung wird hier durch die Ringmethode nach DuNoüy bei 25°C bestimmt.

Bei den Tensiden handelt es sich insbesondere um nicht-ionische Tenside, anionische Tenside, kationische Tenside und amphotere Tenside, wobei der Begriff "amphoteres Tensid" zwitterionischen Tenside umfasst.

Es sind erfindungsgemäße Formulierungen bevorzugt, die dadurch gekennzeichnet sind, dass das Tensid ausgewählt ist aus der Gruppe umfassend, bevorzugt bestehend aus:
anionische Tenside, nicht-ionische Tenside und amphotere Tenside, wobei anionische Tenside und amphotere Tenside besonders bevorzugt sind.

Enthält die erfindungsgemäße Formulierung ein anionisches Tensid, sind insbesondere erfindungsgemäße Formulierungen bevorzugt, die dadurch gekennzeichnet sind, dass das anionische Tensid ausgewählt ist aus der Gruppe umfassend, bevorzugt bestehend aus:

Alkylsulfate und Alkylethersulfate in Form ihrer Alkali-, Erdalkali-, Ammonium- oder Alkanolammoniumsalze,
Alkylphosphate in Form ihrer Alkali-, Ammonium- oder Alkanolammoniumsalze,
Alkylethercarboxylate in Form ihrer Alkali-oder Ammoniumsalze,
Acylisethionate und Acylalkylisethionate in Form ihrer Alkali- oder Ammoniumsalze,
Acylsarkosinate in Form ihrer Alkali- oder Ammoniumsalze,
Sulfosuccinate in Form ihrer Alkali- oder Ammoniumsalze und
Acylglutamate in Form ihrer Alkali- oder Ammoniumsalze,

Acylglycinate in Form ihrer Alkali- oder Ammoniumsalze,
Acylalaninate in Form ihrer Alkali- oder Ammoniumsalze.

wobei Alkylsulfate und Alkylethersulfate besonders bevorzugt sind.

Enthält die erfindungsgemäße Formulierung ein amphoteres Tensid, sind insbesondere erfindungsgemäße Formulierungen bevorzugt, die dadurch gekennzeichnet sind, dass das amphotere Tensid ausgewählt ist aus der Gruppe umfassend, bevorzugt bestehend aus:

Betaine, Amphoacetate und Amphopropionate, N-Alkyl-N,N-dimethylammoniumglycinate, beispielsweise ein Alkyldimethylammoniumglycinat enthaltend Alkylketten mit 8 bis 18 C-Atomen, bevorzugt 12 bis 16 C-Atomen, N-Acylaminopropyl-N,N-dimethylammoniumglycinate, beispielsweise ein Acylaminopropyldimethylammoniumglycinat enthaltend Acylreste mit 8 bis 18 C-Atomen,
2-Alkyl-3-carboxylmethyl-3-hydroxyethylimidazoline mit jeweils 8 bis 18 C-Atomen in der Alkyl- oder Acylgruppe sowie ein Acylaminoethylhydroxyethylcarboxymethylglycinat mit 8 bis 18 C-Atomen in der Acylgruppe,
Verbindungen, die außer einer C8- bis C18-Alkyl- oder -Acylgruppe im Molekül mindestens eine freie Aminogruppe und mindestens eine -COOH- oder -$SO_3$H- Gruppe enthalten und zur Ausbildung innerer Salze befähigt sind, wie zum Beispiel N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 18 C-Atomen in der Alkylgruppe, N-Acylalkylaminopropionat,
Acylaminoethylaminopropionat, jeweils enthaltend Acyl-/Alkylreste mit 8 bis 18 C-Atomen, und C12/18-Acylsarcosin, wobei N-Acylaminopropyl-N,N-dimethylammoniumglycinate enthaltend Acylreste mit 8 bis 18 C-Atomen besonders bevorzugt sind.

Enthält die erfindungsgemäße Formulierung ein anionisches und/oder ein amphoteres Tensid, sind insbesondere erfindungsgemäße Formulierungen bevorzugt, die dadurch gekennzeichnet sind, dass sie 0,1 bis 5 %, bevorzugt 0,5 bis 5 % Salz, insbesondere ausgewählt aus Alkali- und Erdalkalihalogeniden, enthalten, wobei Kochsalz erfindungsgemäß bevorzugt ist.

In einer weiteren bevorzugte Ausführungsform der erfindungsgemäßen Formulierungen sind in der Formulierung neben Wasser und erfindungsgemäßem Polyglycerinalkoxylatester mindestens ein gegebenenfalls alkoxylierter, insbesondere ethoxylierter, Alkohol, welcher bevorzugt bei 1 bar einen Siedepunkt von über 150 °C aufweist, enthalten. Dieser ist bevorzugt ausgewählt aus zwei- und mehrwertigen Alkoholen und Fettalkoholethoxylaten, besonders bevorzugt ausgewählt aus Glycerin, 1,2- und 1,3-Propandiol, Dipropylenglykol sowie ethoxyliertem Laurylalkohol.
Anstelle des oder zusätzlich zu dem gegebenenfalls alkoxylierten Alkohol kann diese weitere bevorzugte erfindungsgemäße Formulierung mindestens einen gegebenenfalls alkoxylierten, insbesondere ethoxylierten, Carbonsäureester enthalten. Diese sind bevorzugt ausgewählt aus Fettsäureestern, insbesondere Polyolethoxylatfettsäureestern, besonders bevorzugt Glycerinethoxylatfettsäureestern, die im Mittel 5 bis 10 Ethylenglykol-Einheiten besitzen.
Im Zusammenhang mit dieser weiteren bevorzugten Ausführungsform enthaltend mindestens einen gegebenenfalls alkoxylierten Alkohol und/oder mindestens einen gegebenenfalls alkoxylierten Carbonsäureester ist einen bevorzugte alternative erfindungsgemäße Formulierung dadurch gekennzeichnet, dass sie die erfindungsgemäßen Polyglycerinalkoxylatester und/oder die nach dem erfindungsgemäßen Verfahren erhältlichen Polyglycerinalkoxylatester in einer Menge von 30 bis 70 Gew.-% bezogen auf die Gesamtformulierung enthält.

Wie oben bereits ausgeführt ist ein weiterer Gegenstand der vorliegenden Erfindung die Verwendung der erfindungsgemäßen Polyglycerinalkoxylatester und der nach dem erfindungsgemäßen Verfahren erhältlichen Polyglycerinalkoxylatester als Verdicker von Formulierungen, insbesondere von den erfindungsgemäßen Formulierungen.
Ebenso ist die Verwendung der erfindungsgemäßen Polyglycerinalkoxylatester und/oder der nach dem erfindungsgemäßen Verfahren erhältlichen Polyglycerinalkoxylatester und/oder der erfindungsgemäßen Formulierungen zur Hautpflege Gegenstand der vorliegenden Erfindung. Ebenso ist die Verwendung der erfindungsgemäßen Polyglycerinalkoxylatester und/oder der nach dem erfindungsgemäßen Verfahren erhältlichen Polyglycerinalkoxylatester zur Schaumstabilisierung von Formulierungen, insbesondere von den erfindungsgemäßen Formulierungen, Gegenstand der vorliegenden Erfindung.
Ebenso ist die Verwendung der erfindungsgemäßen Polyglycerinalkoxylatester und/oder der nach dem erfindungsgemäßen Verfahren erhältlichen Polyglycerinalkoxylatester zur Reduktion der Reizwirkung von kosmetischen Formulierungen auf insbesondere Haut und/oder Augen Gegenstand der vorliegenden Erfindung.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Beispiele:

Beispiel 1: Herstellung von Polyglycerinestern

*1.1 Herstellung von Polyglycerin-6-pentastearat*

Unter Stickstoffatmosphäre wurden 90,0 g Polyglycerin-6 der Fa. Spiga Nord (Hydroxylzahl = 976 mg KOH / g) mit 256 g Stearinsäure (4,5 Moläquiv.) bei 240 °C gerührt bis eine Säurezahl von < 2 mg KOH / g erreicht war. Das im Verlauf der Reaktion gebildete Wasser wurde kontinuierlich abdestilliert. Nach Abkühlung auf Raumtemperatur lag das Reaktionsprodukt als gelblicher Feststoff vor.

*1.2 Herstellung von Polyglycerin-6-penta(stearat/palmitat)*

Unter Stickstoffatmosphäre wurden 216 g Polyglycerin-6 der Fa. Daicel (Hydroxylzahl = 973 mg KOH / g) mit einem Gemisch aus 300 g Stearinsäure (2,2 Moläquiv.) und 284 g Palmitinsäure (2,3 Moläquiv.) bei 240 °C gerührt bis eine Säurezahl von < 2 mg KOH / g erreicht war. Das im Verlauf der Reaktion gebildete Wasser wurde kontinuierlich abdestilliert. Nach Abkühlung auf Raumtemperatur lag das Reaktionsprodukt als gelblicher Feststoff vor.

*1.3 Herstellung von Polyglycerin-6-pentabehenat*

Unter Stickstoffatmosphäre wurden 76,5 g Polyglycerin-6 der Fa. Spiga Nord (Hydroxylzahl = 976 mg KOH / g) mit 260 g Behensäure (4,5 Moläquiv.) bei 240 °C gerührt bis eine Säurezahl von < 2 mg KOH / g erreicht war. Das im Verlauf der Reaktion gebildete Wasser wurde kontinuierlich abdestilliert. Nach Abkühlung auf Raumtemperatur lag das Reaktionsprodukt als gelblicher Feststoff vor.

*1.4 Herstellung von Polyglycerin-3-tetra(stearat/palmitat)*

Unter Stickstoffatmosphäre wurden 62 g Polyglycerin-3 der Fa. Solvay (Hydroxylzahl = 1155 mg KOH / g) mit einem Gemisch aus 139 g Stearinsäure (2,0 Moläquiv.) und 131 g Palmitinsäure (2,0 Moläquiv.) bei 240 °C gerührt bis eine Säurezahl von < 2 mg KOH / g erreicht war. Das im Verlauf der Reaktion gebildete Wasser wurde kontinuierlich abdestilliert. Nach Abkühlung auf Raumtemperatur lag das Reaktionsprodukt als gelblicher Feststoff vor.

*1.5 Herstellung von Polyglycerin-6-hexaoleat*

Unter Stickstoffatmosphäre wurden 75 g Polyglycerin-6 der Fa. Daicel (Hydroxylzahl = 973 mg KOH / g) mit 280 g Ölsäure (6,0 Moläquiv.) bei 240 °C gerührt bis eine Säurezahl von < 2 mg KOH / g erreicht war. Das im Verlauf der Reaktion gebildete Wasser wurde kontinuierlich abdestilliert. Nach Abkühlung auf Raumtemperatur lag das Reaktionsprodukt als gelblicher Feststoff vor.

*1.6 Herstellung von Polyglycerin-6-pentaoleat*

Unter Stickstoffatmosphäre wurden 90 g Polyglycerin-6 der Fa. Daicel (Hydroxylzahl = 973 mg KOH / g) mit 280 g Ölsäure (5,0 Moläquiv.) bei 240 °C gerührt bis eine Säurezahl von < 2 mg KOH / g erreicht war. Das im Verlauf der Reaktion gebildete Wasser wurde kontinuierlich abdestilliert. Nach Abkühlung auf Raumtemperatur lag das Reaktionsprodukt als gelblicher Feststoff vor.

*1.7 Herstellung von Polyglycerin-6-tetra(stearat/palmitat)*

Unter Stickstoffatmosphäre wurden 90 g Polyglycerin-6 der Fa. Daicel (Hydroxylzahl = 973 mg KOH / g) mit einem Gemisch aus 108 g Stearinsäure (1,9 Moläquiv.) und 102 g Palmitinsäure (2,0 Moläquiv.) bei 240 °C gerührt bis eine Säurezahl von < 2 mg KOH / g erreicht war. Das im Verlauf der Reaktion gebildete Wasser wurde kontinuierlich abdestilliert. Nach Abkühlung auf Raumtemperatur lag das Reaktionsprodukt als gelblicher Feststoff vor.

Beispiel 2: Herstellung von Polyglycerinalkoxylatestern

Bei den Polyglycerinalkoxylatestern A bis H handelt es sich um erfindungsgemäße Beispiele, bei dem Polyglycerinalkoxylatester I um ein nicht-erfindungsgemäßes Vergleichsbeispiel.

a.) mittels Alkoxylierung von Polyglycerinestern

*2.1 Herstellung des Polyglycerin-6-basierten Polyglycerinalkoxylatesters A mit 450 Moläquivalenten EO und 4,5 Moläquivalenten Stearinsäure (erfindungsgemäß)*

In einem 5 Liter Autoklaven wurden 300 g des Polyglycerinesters aus Beispiel 1.1 zusammen mit 3 Mol-% wässriger KOH-Lösung (45%ig) vorgelegt. Zum Inertisieren wurde der Reaktor bis 3 bar mit Stickstoff beaufschlagt und anschließend auf Normaldruck entspannt. Der Vorgang wurde noch zweimal wiederholt. Unter Rühren wurde der Reaktorinhalt auf 150 °C erhitzt und auf ca. 20 mbar evakuiert, um das Wasser aus dem Katalyseschritt destillativ zu entfernen. Dann wurden bei einer Temperatur von 170°C 1638 g Ethylenoxid (EO) dergestalt angelagert, dass der Druck im Reaktor nicht über 4 bar Überdruck anstieg. Nach beendeter Dosage wurde solange gewartet, bis der Druck nicht weiter sank. Nach Erreichen dieser Druckkonstanz wurde noch eine einstündige Nachreaktion bei 170°C durchgeführt. Anschließend wurde das Reaktionsgemisch auf 95°C abgekühlt und unter ca. 15-minütigem Rühren durch Anlegen eines Drucks ($p < 20$ mbar) desodoriert, um Spuren an nicht umgesetztem Alkylenoxid zu entfernen. Dann wurde das Produkt teilweise abgelassen. An das im Reaktor verbliebene Intermediat (908 g) wurden anschließend bei 150°C weitere 959 g EO angelagert. Nach Erreichen der Druckkonstanz und einer finalen 1 h Nachreaktion wurde das Produkt wie zuvor bei 95°C desodoriert.
Es wurde ein bei RT festes, farbloses bis gelbliches Produkt erhalten, dass eine OH-Zahl von 13,6 mg KOH/g und eine Verseifungszahl VZ von 13 mg KOH/g aufwies sowie eine SZ von 0,1 mg KOH/g. Laut GPC betrug $M_w$ = 14522 g/mol, $M_n$ = 8225 g/mol und der PDI betrug 1,77.

*2.2 Herstellung des Polyglycerin-6-basierten Polyglycerinalkoxylatesters B mit 450 Moläquivalenten EO und 4,5 Moläquivalenten C16/C18-Fettsäure (erfindungsgemäß)*

In einem 5 Liter Autoklaven wurden 408 g des Polyglycerinesters aus Beispiel 1.2 zusammen mit 3 Mol-% wässriger KOH-Lösung (45%ig) vorgelegt. Zum Inertisieren wurde der Reaktor bis 3 bar mit Stickstoff beaufschlagt und anschließend auf Normaldruck entspannt. Der Vorgang wurde noch zweimal wiederholt. Unter Rühren wurde der Reaktorinhalt auf 150 °C erhitzt und auf ca. 20 mbar evakuiert, um das Wasser aus dem Katalyseschritt destillativ zu entfernen. Dann wurden bei einer Temperatur von 170°C 2125 g EO dergestalt angelagert, dass der Druck im Reaktor nicht über 4 bar Überdruck anstieg. Nach beendeter Dosage wurde solange gewartet, bis der Druck nicht weiter sank. Nach Erreichen dieser Druckkonstanz wurde noch eine einstündige Nachreaktion bei 170°C durchgeführt. Anschließend wurde das Reaktionsgemisch auf 95°C abgekühlt und unter ca. 15-minütigem Rühren durch Anlegen eines Drucks ($p < 20$ mbar) desodoriert, um Spuren an nicht umgesetztem Alkylenoxid zu entfernen. Dann wurde das Produkt teilweise abgelassen. An das im Reaktor verbliebene Intermediat (661 g) wurden anschließend bei 130°C weitere 692 g EO angelagert. Nach Erreichen der Druckkonstanz und einer finalen 1 h Nachreaktion wurde das Produkt wie zuvor bei 95°C desodoriert.
Es wurde ein festes, farbloses bis gelbliches Produkt erhalten, dass eine OH-Zahl von 13,7 mg KOH/g und eine Verseifungszahl VZ von 15 mg KOH/g aufwies sowie eine SZ von 0,1 mg KOH/g. Laut GPC betrug $M_w$ = 17059 g/mol, $M_n$ = 7085 g/mol und der PDI betrug 2,11.

*2.3 Herstellung des Polyglycerin-6-basierten Polyglycerinalkoxylatesters C mit 450 Moläquivalenten EO und 4,5 Moläquivalenten Behensäure (erfindungsgemäß)*

In einem 5 Liter Autoklaven wurden 273 g des Polyglycerinesters aus Beispiel 1.3 zusammen mit 3 Mol-% wässriger KOH-Lösung (45%ig) vorgelegt. Zum Inertisieren wurde der Reaktor bis 3 bar mit Stickstoff beaufschlagt und anschließend auf Normaldruck entspannt. Der Vorgang wurde noch zweimal wiederholt. Unter Rühren wurde der Reaktorinhalt auf 150 °C erhitzt und auf ca. 20 mbar evakuiert, um das Wasser aus dem Katalyseschritt destillativ zu entfernen. Dann wurden bei einer Temperatur von 170°C 1294 g EO dergestalt angelagert, dass der Druck im Reaktor nicht über 4 bar Überdruck anstieg. Nach beendeter Dosage wurde solange gewartet, bis der Druck nicht weiter sank. Nach Erreichen dieser Druckkonstanz wurde noch eine einstündige Nachreaktion bei 170°C durchgeführt. Anschließend wurde das Reaktionsgemisch auf 95°C abgekühlt und unter ca. 15-minütigem Rühren durch Anlegen eines Drucks ($p < 20$ mbar) desodoriert, um Spuren an nicht umgesetztem Alkylenoxid zu entfernen. Dann wurde das Produkt teilweise abgelassen. An das im Reaktor verbliebene Intermediat (573 g) wurden anschließend bei 170°C weitere 591 g EO angelagert. Nach Erreichen der Druckkonstanz und einer finalen 1 h Nachreaktion wurde das Produkt wie zuvor bei 95°C desodoriert.
Es wurde ein festes, farbloses bis gelbliches Produkt erhalten, dass eine OH-Zahl von 14,2 mg KOH/g und eine Verseifungszahl VZ von 14 mg KOH/g aufwies sowie eine SZ von < 0,1 mg KOH/g. Laut GPC betrug $M_w$ = 13594 g/mol, $M_n$ = 8350 g/mol und der PDI betrug 1,63.

*2.4 Herstellung des Polyglycerin-3-basierten Polyglycerinalkoxylatesters E mit 300 Moläquivalenten EO und 4,0 Moläquivalenten C16/C18-Fettsäure (erfindungsgemäß)*

In einem 5 Liter Autoklaven wurden 286,5 g des Polyglycerinesters aus Beispiel 1.4 zusammen mit 2 Mol-% wässriger KOH-Lösung (45%ig) vorgelegt. Zum Inertisieren wurde der Reaktor bis 3 bar mit Stickstoff beaufschlagt und anschließend auf Normaldruck entspannt. Der Vorgang wurde noch zweimal wiederholt. Unter Rühren wurde der Reaktorinhalt auf 150 °C erhitzt und auf ca. 20 mbar evakuiert, um das Wasser aus dem Katalyseschritt destillativ zu entfernen. Dann wurden bei einer Temperatur von 170°C 985 g EO dergestalt angelagert, dass der Druck im Reaktor nicht über 4 bar Überdruck anstieg. Nach beendeter Dosage wurde solange gewartet, bis der Druck nicht weiter sank. Nach Erreichen dieser Druckkonstanz wurde noch eine einstündige Nachreaktion bei 170°C durchgeführt. Anschließend wurde das Reaktionsgemisch auf 95°C abgekühlt und unter ca. 15-minütigem Rühren durch Anlegen eines Drucks ($p < 20$ mbar) desodoriert, um Spuren an nicht umgesetztem Alkylenoxid zu entfernen. Dann wurde das Produkt teilweise abgelassen. An das im Reaktor verbliebene Intermediat (712 g) wurden anschließend bei 170°C weitere 827 g EO angelagert. Nach Erreichen der Druckkonstanz und einer finalen 1 h Nachreaktion wurde das Produkt wie zuvor bei 95°C desodoriert.
Es wurde ein festes, farbloses bis gelbliches Produkt erhalten, dass eine OH-Zahl von 10,4 mg KOH/g und eine Verseifungszahl VZ von 11 mg KOH/g aufwies sowie eine SZ von 0,1 mg KOH/g. Laut GPC betrug $M_w$ = 11607 g/mol, $M_n$ = 7655 g/mol und der PDI betrug 1,52.

*2.5 Herstellung des Polyglycerin-6-basierten Polyglycerinalkoxylatesters F mit 400 Moläquivalenten EO und 6,0 Moläquivalenten Ölsäure (erfindungsgemäß)*

In einem 5 Liter Autoklaven wurden 328,6 g des Polyglycerinesters aus Beispiel 1.5 zusammen mit 3 Mol-% wässriger KOH-Lösung (45%ig) vorgelegt. Zum Inertisieren wurde der Reaktor bis 3 bar mit Stickstoff beaufschlagt und anschließend auf Normaldruck entspannt. Der Vorgang wurde noch zweimal wiederholt. Unter Rühren wurde der Reaktorinhalt auf 150 °C erhitzt und auf ca. 20 mbar evakuiert, um das Wasser aus dem Katalyseschritt destillativ zu entfernen. Dann wurden bei einer Temperatur von 170°C 1469 g EO dergestalt angelagert, dass der Druck im Reaktor nicht über 4 bar Überdruck anstieg. Nach beendeter Dosage wurde solange gewartet, bis der Druck nicht weiter sank. Nach Erreichen dieser Druckkonstanz wurde noch eine einstündige Nachreaktion bei 170°C durchgeführt. Anschließend wurde das Reaktionsgemisch auf 95°C abgekühlt und unter ca. 15-minütigem Rühren durch Anlegen eines Drucks ($p < 20$ mbar) desodoriert, um Spuren an nicht umgesetztem Alkylenoxid zu entfernen. Dann wurde das Produkt teilweise abgelassen. An das im Reaktor verbliebene Intermediat (970 g) wurden anschließend bei 170°C weitere 792 g EO angelagert. Nach Erreichen der Druckkonstanz und einer finalen 1 h Nachreaktion wurde das Produkt wie zuvor bei 95°C desodoriert.
Es wurde ein festes, farbloses bis gelbliches Produkt erhalten, dass eine OH-Zahl von 12,0 mg KOH/g und eine Verseifungszahl VZ von 14 mg KOH/g aufwies sowie eine SZ von 0,1 mg KOH/g. Laut GPC betrug $M_w$ = 14427 g/mol, $M_n$ = 8944 g/mol und der PDI betrug 1,61.

*2.6 Herstellung des Polyglycerin-6-basierten Polyglycerinalkoxylatesters G mit 500 Moläquivalenten EO und 5,0 Moläquivalenten Ölsäure (erfindungsgemäß)*

In einem 5 Liter Autoklaven wurden 309,1 g des Polyglycerinesters aus Beispiel 1.6 zusammen mit 3 Mol-% wässriger KOH-Lösung (45%ig) vorgelegt. Zum Inertisieren wurde der Reaktor bis 3 bar mit Stickstoff beaufschlagt und anschließend auf Normaldruck entspannt. Der Vorgang wurde noch zweimal wiederholt. Unter Rühren wurde der Reaktorinhalt auf 150 °C erhitzt und auf ca. 20 mbar evakuiert, um das Wasser aus dem Katalyseschritt destillativ zu entfernen. Dann wurden bei einer Temperatur von 170°C 1587 g EO dergestalt angelagert, dass der Druck im Reaktor nicht über 4 bar Überdruck anstieg. Nach beendeter Dosage wurde solange gewartet, bis der Druck nicht weiter sank. Nach Erreichen dieser Druckkonstanz wurde noch eine einstündige Nachreaktion bei 170°C durchgeführt. Anschließend wurde das Reaktionsgemisch auf 95°C abgekühlt und unter ca. 15-minütigem Rühren durch Anlegen eines Drucks ($p < 20$ mbar) desodoriert, um Spuren an nicht umgesetztem Alkylenoxid zu entfernen. Dann wurde das Produkt teilweise abgelassen. An das im Reaktor verbliebene Intermediat (805 g) wurden anschließend bei 170°C weitere 1011 g EO angelagert. Nach Erreichen der Druckkonstanz und einer finalen 1 h Nachreaktion wurde das Produkt wie zuvor bei 95°C desodoriert.
Es wurde ein festes, farbloses bis gelbliches Produkt erhalten, dass eine OH-Zahl von 13,9 mg KOH/g und eine Verseifungszahl VZ von 14 mg KOH/g aufwies sowie eine SZ von 0,1 mg KOH/g. Laut GPC betrug $M_w$ = 25490 g/mol, $M_n$ = 7040 g/mol und der PDI betrug 3,62.

*2.7 Herstellung des Polyglycerin-3-basierten Polyglycerinalkoxylatesters H mit 400 Moläquivalenten EO und 4,0 Moläquivalenten C16/C18-Fettsäure (erfindungsgemäß)*

In einem 5 Liter Autoklaven wurden 293,7 g des Polyglycerinesters aus Beispiel 1.7 zusammen mit 2 Mol-% wässriger KOH-Lösung (45%ig) vorgelegt. Zum Inertisieren wurde der Reaktor bis 3 bar mit Stickstoff beaufschlagt und anschließend auf Normaldruck entspannt. Der Vorgang wurde noch zweimal wiederholt. Unter Rühren wurde der Reaktorinhalt auf 150 °C erhitzt und auf ca. 20 mbar evakuiert, um das Wasser aus dem Katalyseschritt destillativ zu entfernen. Dann wurden bei einer Temperatur von 170°C 1744 g EO dergestalt angelagert, dass der Druck im Reaktor nicht über 4 bar Überdruck anstieg. Nach beendeter Dosage wurde solange gewartet, bis der Druck nicht weiter sank. Nach Erreichen dieser Druckkonstanz wurde noch eine einstündige Nachreaktion bei 170°C durchgeführt. Anschließend wurde das Reaktionsgemisch auf 95°C abgekühlt und unter ca. 15-minütigem Rühren durch Anlegen eines Drucks (p < 20 mbar) desodoriert, um Spuren an nicht umgesetztem Alkylenoxid zu entfernen. Dann wurde das Produkt teilweise abgelassen. An das im Reaktor verbliebene Intermediat (1131 g) wurden anschließend bei 170°C weitere 967 g EO angelagert. Nach Erreichen der Druckkonstanz und einer finalen 1 h Nachreaktion wurde das Produkt wie zuvor bei 95°C desodoriert.
Es wurde ein festes, farbloses bis gelbliches Produkt erhalten, dass eine OH-Zahl von 13,2 mg KOH/g und eine Verseifungszahl VZ von 14 mg KOH/g aufwies sowie eine SZ von 0,1 mg KOH/g. Laut GPC betrug $M_w$ = 20394 g/mol, $M_n$ = 6827 g/mol und der PDI betrug 2,99.

b.) mittels Veresterung von Polyglycerinethoxylaten

*2.8 Herstellung des Polyglycerin-3-basierten Polyglycerinalkoxylatesters D mit 300 Moläquivalenten EO und 3,0 Moläquivalenten Ölsäure (erfindungsgemäß)*

2.8.1 Alkoxylierung: In einem 5 Liter Autoklaven wurden 104,7 g Polyglycerin-3 (Fa. Solvay) zusammen mit 2 Mol-% wässriger KOH-Lösung (45%ig) vorgelegt. Zum Inertisieren wurde der Reaktor bis 3 bar mit Stickstoff beaufschlagt und anschließend auf Normaldruck entspannt. Der Vorgang wurde noch zweimal wiederholt. Unter Rühren wurde der Reaktorinhalt auf 150 °C erhitzt und auf ca. 20 mbar evakuiert, um das Wasser aus dem Katalyseschritt destillativ zu entfernen. Dann wurden bei einer Temperatur von 170°C 3777 g EO dergestalt angelagert, dass der Druck im Reaktor nicht über 4 bar Überdruck anstieg. Nach beendeter Dosage wurde solange gewartet, bis der Druck nicht weiter sank. Nach Erreichen dieser Druckkonstanz wurde noch eine einstündige Nachreaktion bei 170°C durchgeführt. Anschließend wurde das Reaktionsgemisch auf 95°C abgekühlt und unter ca. 15-minütigem Rühren durch Anlegen eines Drucks (p < 20 mbar) desodoriert, um Spuren an nicht umgesetztem Alkylenoxid zu entfernen. Dann wurde das Produkt teilweise abgelassen. An das im Reaktor verbliebene Intermediat (2369 g) wurden anschließend bei 170°C weitere 1148 g EO angelagert. Nach Erreichen der Druckkonstanz und einer finalen 1 h Nachreaktion wurde das Produkt wie zuvor bei 95°C desodoriert.
Es wurde ein festes, farbloses bis gelbliches Produkt erhalten, dass eine OH-Zahl von 22,0 mg KOH/g aufwies sowie eine SZ von 0,2 mg KOH/g. Laut GPC betrug $M_w$ = 11761 g/mol, $M_n$ = 10359 g/mol und der PDI betrug 1,14.
2.8.2 Veresterung: Unter Stickstoffatmosphäre und auf 50 mbar vermindertem Druck wurden 255 g des Ethoxylats aus Teilbeispiel 2.8.1 mit 16,7 g Ölsäure (3,0 Moläquiv.) bei 140 °C gerührt bis eine Säurezahl von < 3 mg KOH / g erreicht war. Das im Verlauf der Reaktion gebildete Wasser wurde kontinuierlich abdestilliert. Nach Abkühlung auf Raumtemperatur lag das Reaktionsprodukt als farbloser Feststoff vor und wies eine Säurezahl von 2,6 mg KOH / g, eine Hydroxylzahl von 13 mg KOH / g und eine Verseifungszahl von 18 mg KOH / g auf.

*2.9 Herstellung des Polyglycerin-3-basierten Polyglycerinalkoxylatesters I mit 250 Moläquivalenten EO und 2,0 Moläquivalenten Ölsäure (nicht erfindungsgemäß)*

2.9.1 Alkoxylierung: In einem 5 Liter Autoklaven wurden 167,1 g Polyglycerin-3 (Fa. Solvay) zusammen mit 2 Mol-% wässriger KOH-Lösung (45%ig) vorgelegt. Zum Inertisieren wurde der Reaktor bis 3 bar mit Stickstoff beaufschlagt und anschließend auf Normaldruck entspannt. Der Vorgang wurde noch zweimal wiederholt. Unter Rühren wurde der Reaktorinhalt auf 150 °C erhitzt und auf ca. 20 mbar evakuiert, um das Wasser aus dem Katalyseschritt destillativ zu entfernen.
Dann wurden bei einer Temperatur von 170°C 3012 g EO dergestalt angelagert, dass der Druck im Reaktor nicht über 4 bar Überdruck anstieg. Nach beendeter Dosage wurde solange gewartet, bis der Druck nicht weiter sank. Nach Erreichen dieser Druckkonstanz wurde noch eine einstündige Nachreaktion bei 170°C durchgeführt. Anschließend wurde das Reaktionsgemisch auf 95°C abgekühlt und unter ca. 15-minütigem Rühren durch Anlegen eines Drucks (p < 20 mbar) desodoriert, um Spuren an nicht umgesetztem Alkylenoxid zu entfernen. Dann wurde das Produkt teilweise abgelassen. An das im Reaktor verbliebene Intermediat (653 g) wurden anschließend bei 170°C weitere 692 g EO

angelagert. Nach Erreichen der Druckkonstanz und einer finalen 1 h Nachreaktion wurde das Produkt wie zuvor bei 95°C desodoriert.
Es wurde ein festes, farbloses bis gelbliches Produkt erhalten, dass eine OH-Zahl von 15,3 mg KOH/g aufwies sowie eine SZ von -0,15 mg KOH/g. Laut GPC betrug $M_w$ = 15330 g/mol, $M_n$ = 12720 g/mol und der PDI betrug 1,21.

2.9.2 Veresterung: Unter Stickstoffatmosphäre wurden 217 g des Ethoxylats aus Teilbeispiel 2.9.1 mit 7,30 g Ölsäure (2,0 Moläquiv.) bei 140 °C und 50 mbar gerührt bis eine Säurezahl von < 3 mg KOH / g erreicht war. Das im Verlauf der Reaktion gebildete Wasser wurde kontinuierlich abdestilliert. Nach Abkühlung auf Raumtemperatur lag das Reaktionsprodukt als farbloser Feststoff vor und wies eine Säurezahl von 1,7 mg KOH / g, eine Hydroxylzahl 10 mg KOH / g und eine Verseifungszahl von 10 mg KOH / g auf.

Beispiel 3: Anwendungsbeispiele:

*Beispiel 3.1: Verbesserte Verdickungsleistung der erfindungsgemäßen Polyglycerinalkoxylatester im Vergleich zu nicht erfindungsgemäßen Ethoxylatestern in wässrigen Tensidlösungen mit marktüblicher Tensidkonzentration*

Die verdickende Wirkung der hergestellten erfindungsgemäßen Polyglycerinalkoxylatesters wurden im Vergleich zu nicht-erfindungsgemäßen, selbst hergestellten Polyglycerinalkoxylatestern sowie im Vergleich zu kommerziell erhältlichen Verdickern in kosmetischen Formulierungen evaluiert. Die Bestandteile der Formulierungen sind in den Zusammensetzungen in Form der allgemein anerkannten INCI-Nomenklatur unter Verwendung der englischen Begriffe benannt. Die Prozentangaben verstehen sich als Gewichtsprozent.

Als kommerziell erhältliche Verdicker wurden die folgenden eingesetzt:

1. REWODERM® LI S 80:

   Standardverdicker auf Basis Glycerinethoxylatester,
   INCI: PEG-200 Hydrogenated Glyceryl Palmate (and) PEG-7 Glyceryl Cocoate

2. ANTIL® 200:

   Standardverdicker auf Basis Glycerinethoxylatester,

   INCI: PEG-200 Hydrogenated Glyceryl Palmate (and) PEG-7 Glyceryl Cocoate

3. ANTIL® 120 Plus:

   Standardverdicker auf Basis Methylglucoseethoxylatester,

   INCI: PEG-120 Methyl Glucose Dioleate

4. Genapol® LT:

   Standardverdicker auf Basis Diglycerinethoxylatester,

   INCI: PEG-150 Polyglyceryl-2 Tristearate (and) Laureth-3 (and) Dipropylene Glycol

Um die Verdickungsleistung der erfindungsgemäßen Polyglycerinalkoxylatesters zu untersuchen, wurden definierte Mengen dieser Produkte bei Raumtemperatur durch Rühren in tensidische Systeme eingearbeitet und anschließend die Viskositäten gemessen.
In Tabelle 1 wird die verbesserte Verdickungsleistung der erfindungsgemäßen Polyglycerinalkoxylatestern der Verdickungsleistung marktüblicher Ethoxylatester gegenübergestellt:

Tabelle 1:

| Gemessene Viskositäten in mPa s (Brookfield-Viskosimeter, 25°C) einer wässrigen Tensidlösung enthaltend 12 Gew.-% einer Mischung aus Sodium Laureth Sulfate und Cocamidopropylbetain (Gewichtsverhältnis 9 zu 3) sowie 0.7% Natriumchlorid und Verdickungsmittel in der angegebenen Menge; eingestellt auf pH 5.5 | | | | | | |
|---|---|---|---|---|---|---|
| Verdickerkonzentration →<br>Eingesetzter Verdicker ↓ | 0.3 Gew.% | 0.4 Gew. % | 0.5 Gew. % | 0.6 Gew. % | 0.7 Gew. % | 0.8 Gew. % |
| Polyglycerinalkoxylatester A | 6133 | -- | 47840 | -- | 85333 | -- |
| Polyglycerinalkoxylatester B | -- | 13387 | 20967 | -- | -- | -- |
| Polyglycerinalkoxylatester C | -- | 13867 | 20480 | -- | -- | -- |
| Polyglycerinalkoxylatester D | -- | 5824 | 13067 | 16053 | -- | -- |
| Polyglycerinalkoxylatester E | -- | -- | 9088 | 14133 | -- | -- |
| Polyglycerinalkoxylatester F | -- | -- | 8309 | 13813 | -- | -- |
| Polyglycerinalkoxylatester G | -- | -- | 6905 | 10144 | -- | -- |
| Polyglycerinalkoxylatester H | -- | -- | 13707 | 19998 | -- | -- |
| Polyglycerinalkoxylatester I (nicht erfindungsgemäß) | -- | -- | -- | -- | -- | 300 |
| PEG-200 Hydrogenated Glyceryl Palmate; PEG-7 Glyceryl Cocoate (REWODERM® LI S 80) (nicht erfindungsgemäß) | -- | -- | 75 | -- | -- | 149 |
| PEG-200 Hydrogenated Glyceryl Palmate; PEG-7 Glyceryl Cocoate (ANTIL® 200) (nicht erfindungsgemäß) | 746 | 1792 | 3676 | 5451 | 8064 | 24213 |
| PEG-120 Methyl Glucose Dioleate (ANTIL® 120 Plus) (nicht erfindungsgemäß) | 3504 | 4128 | 6816 | 9707 | 22720 | 17293 |
| PEG-150 Polyglyceryl-2 Tristearate (and) Laureth-3 (and) Dipropylene Glycol (Genapol® LT) (nicht erfindungsgemäß) | | | 2613 | | 7851 | |

Anhand der in Tabelle 1 dargestellten Ergebnisse wird ersichtlich, dass bei Einsatz gleicher Substanzmengen mit den erfindungsgemäßen Polyglycerinalkoxylatestern höher viskose Tensidsysteme erhalten werden als mit nicht-erfindungsgemäßen Polyglycerylethoxylatestern , d. h. die erforderliche Menge an Verdicker zur Erreichung einer bestimmten Zielviskosität ist im Falle der erfindungsgemäßen Polyglycerinalkoxylatester erheblich geringer als im Falle der nicht-erfindungsgemäßen Ethoxylatester.

*Beispiel 3.2: Verbesserte Verdickungsleistung der erfindungsgemäßen Polyglycerinalkoxylatester im Vergleich zu nicht erfindungsgemäßen Ethoxylatestern in wässrigen Tensidlösungen mit niedriger Tensidkonzentration*

Neben den in Beispiel 3.1 gezeigten Verdickungseigenschaften der erfindungsgemäßen Polyglycerinalkoxylatester in wässrigen Tensidlösungen mit marktüblicher Tensidkonzentration wurde die Verdickungsleistung auch in wässrigen tensidischen Lösungen mit niedriger Tensidkonzentration untersucht. Die für die erfindungsgemäßen Polyglycerinalkoxylatester nach Einrühren in das wässrige Tensidsystem gemessenen Viskositäten sind in den Tabellen 2 und 3 den Viskositäten gegenübergestellt, die durch nicht erfindungsgemäßer Ethoxylatester erzielt wurden:

Tabelle 2:

| Gemessene Viskositäten in mPa s (Brookfield-Viskosimeter, 25°C) einer wässrigen Tensidlösung enthaltend 9 Gew.-% einer Mischung aus Sodium Laureth Sulfate und Cocamidopropylbetain (Gewichtsverhältnis 7 zu 2) sowie 0.7% Natriumchlorid und Verdickungsmittel in der angegebenen Menge; eingestellt auf pH 5.5 | | | |
|---|---|---|---|
| Verdickerkonzentration → Eingesetzter Verdicker ↓ | 1.5 Gew.% | 2.0 Gew.% | 2.5 Gew.% |
| Polyglycerinalkoxylatester E | 1419 | | 20220 |
| Polyglycerinalkoxylatester F | 3168 | | 27080 |
| Polyglycerinalkoxylatester G | 2976 | | 23947 |
| Polyglycerinalkoxylatester H | | 19397 | 51040 |
| PEG-200 Hydrogenated Glyceryl Palmate; PEG-7 Glyceryl Cocoate (ANTIL®200) (nicht erfindungsgemäß) | 416 | 1792 | 11220 |
| PEG-120 Methyl Glucose Dioleate (ANTIL® 120 Plus) (nicht erfindungsgemäß) | 832 | 2648 | 11680 |

Tabelle 3:

| Gemessene Viskositäten in mPa s (Brookfield-Viskosimeter, 25°C) einer wässrigen Tensidlösung enthaltend 7 Gew.-% einer Mischung aus Sodium Laureth Sulfate und Cocamidopropylbetain (Gewichtsverhältnis 5 zu 2) sowie 1.5% Natriumchlorid und Verdickungsmittel in der angegebenen Menge; eingestellt auf pH 5.5 | | | | | |
|---|---|---|---|---|---|
| Verdickerkonzentration → Eingesetzter Verdicker ↓ | 0.2 Gew. % | 0.3 Gew. % | 0.5 Gew. % | 1.0 Gew. % | 1.5 Gew. % |
| Polyglycerinalkoxylatester A | 9643 | 24720 | | | |
| Polyglycerinalkoxylatester B | | | 46533 | 96173 | 100000 |
| PEG-200 Hydrogenated Glyceryl Palmate; PEG-7 Glyceryl Cocoate (REWODERM® LI S 80) (nicht erfindungsgemäß) | 32 | 42 | | | |
| PEG-200 Hydrogenated Glyceryl Palmate; PEG-7 Glyceryl Cocoate (ANTIL® 200;) (nicht erfindungsgemäß) | 778 | 1963 | 7232 | 20160 | 36320 |
| PEG-120 Methyl Glucose Dioleate (ANTIL® 120 Plus) (nicht erfindungsgemäß) | 917 | 2453 | 7733 | 14933 | 18427 |
| PEG-150 Polyglyceryl-2 Tristearate (and) Laureth-3 (and) Dipropylene Glycol (Genapol® LT) (nicht erfindungsgemäß) | | 1344 | 5227 | | |

Wie in den Tabellen 2 und 3 gezeigt, können selbst in solchen Tensidsystemen, die sich aufgrund ihres geringen Gehalts an waschaktiven Substanzen nur durch verhältnismäßig hohe Mengen herkömmlicher Verdickungsmittel verdicken lassen, durch verhältnismäßig geringe Mengen der erfindungsgemäßen Verdicker hohe Viskositäten eingestellt werden.

*Beispiel 3.3: Verbesserte Schaumeigenschaften und verbessertes Hautpflegevermögen der erfindungsgemäßen Polyglycerinalkoxylatester im Vergleich zu nicht erfindungsgemäßen Ethoxylatestern in wässrigen Tensidmischungen*

Zur Bewertung der Schaumeigenschaften und Hautpflegeleistung des erfindungsgemäßen Polyglycerinalkoxylatesters A in wässrigen, tensidischen Formulierungen wurde ein sensorischer Handwaschtest im Vergleich zum nicht erfindungsgemäßen Ethoxylatester PEG-200 Hydrogenated Glyceryl Palmate; PEG-7 Glyceryl Cocoate (ANTIL® 200) nach dem Stand der Technik durchgeführt.

Eine Gruppe bestehend aus 10 trainierten Prüfpersonen wusch sich dabei definiert die Hände und bewertete Schaumeigenschaften und Hautgefühl anhand einer Notenskala von 1 (sehr schlecht) bis 5 (sehr gut).
Die Produkte wurden jeweils in einer standardisierten Tensidformulierung getestet, bestehend aus dem Standard-Tensidsystem 9% aktiv Sodium Laureth Sulfate und 3% aktiv Cocamidopropyl Betaine (siehe Tabelle 4).

Tab. 4: Testformulierungen für den Handwaschtest, pH 5,5

| **Formulierungsbeispiele** | **I** | **II** |
|---|---|---|
| Texapon® NSO-IS, BASF Cognis, 28%-ig, (INCI: Sodium Laureth Sulfate) | 32,0% | 32,0% |
| TEGO® Betain F 50, Evonik Industries AG, 38%-ig, (INCI: Cocamidopropyl Betaine) | 8,0% | 8,0% |
| NaCl | 0,7% | 0,7% |
| Zitronensäure | q.s. | q.s. |
| Wasser, demineralisiert | ad 100 | ad 100 |
| Polyglycerinalkoxylatester A ; PEG-7 Glyceryl Cocoate (erfindungsgemäß) | 0,5% | - |
| PEG-200 Hydrogenated Glyceryl Palmate; PEG-7 Glyceryl Cocoate (ANTIL® 200) (nicht erfindungsgemäß) | - | 0,8% |

Unterschiedliche Einsatzkonzentrationen sind der Einstellung einer identischer Viskosität der Formulierungen geschuldet; die sensorischen Testergebnisse sind in Tabelle 5 zusammengefasst.

Tab. 5: Ergebnisse des Handwaschtests

| | **Formulierung I** | **Formulierung II** |
|---|---|---|
| Anschäumbarkeit | **3,05** | 2,85 |
| Schaumcremigkeit | **2,90** | 2,75 |
| Abwaschbarkeit | 3,40 | 3,40 |
| Hautglätte | **2,10** | 1,95 |
| Hautweichheit | **2,45** | 2,20 |
| Hautglätte nach 3 min | **3,80** | 3,65 |
| Hautweichheit nach 3 min. | **3,80** | 3,65 |

Anhand der Testergebnisse in Tabelle 5 wird ersichtlich, dass die erfindungsgemäße Formulierung unter Verwendung des erfindungsgemäßen Polyglycerinalkoxylatesters A überraschenderweise zu einer Verbesserung der Anschäumbarkeit und der Cremigkeit des Schaums führt und die Hautglätte und -Weichheit erhöht.

*Beispiel 3.4: Verbesserte Mildheit von Tensidmischungen enthaltend erfindungsgemäße Polyglycerinalkoxylatester*

Die Mildheit der erfindungsgemäßen Zusammensetzungen wurde *in vitro* mithilfe des Red Blood Cell Tests (RBC-Test) bestimmt, der eine Einschätzung des Reizpotentials von Tensiden und tensidischen Gemischen ermöglicht. Für den Test wird eine Erythrozyten-Suspension verwendet, die aus Schweineblut gewonnen wird. Es werden zwei Werte gemessen: Der $H_{50}$-Wert, der diejenige Menge an Tensid angibt, die notwendig ist, um 50% der Erythrozyten zu zerstören, sowie der DI-Wert, der den Grad der Zerstörung in % (Denaturierung) des Hämoglobins durch das zu untersuchende Tensid oder Tensidgemisch im Verhältnis zu einer Zerstörung durch eine 1%ige Na-Dodecylsulfat-Lösung angibt. Der Quotient aus dem $H_{50}$-(L) und dem DI-Wert (D), der L/D-Wert, wird zur Abschätzung des Reizpotentials der gemessenen Tenside oder tensidischen Gemische herangezogen. Je größer er ist, desto milder ist das Tensid oder Tensidgemisch einzustufen.
Zur Bewertung des Einflusses des erfindungsgemäßen Polyglycerinalkoxylatesters A auf die Mildheit eines Tensidgemisches wurde der L/D-Wert für ein Tensidgemisch enthaltend 7 Gew.-% einer Mischung aus Sodium Laureth Sulfate und Cocamidopropylbetain (Gewichtsverhältnis 5 zu 2) sowie 1.5% Natriumchlorid jeweils einmal mit und einmal ohne den erfindungsgemäßen Polyglycerinalkoxylatester A bestimmt. Die Ergebnisse waren wie nachstehend (siehe Tabelle

6).

Tab. 6: Ergebnisse des Red Blood Cell Test

| Tensidgemisch | | UD-Wert |
|---|---|---|
| III | 5% Sodium Laureth Sulfate, 2% Cocamidopropylbetaine; 1.5% NaCl | 0,5 |
| IV | 5% Sodium Laureth Sulfate, 2% Cocamidopropylbetaine; 1.5% NaCl; 0,1% Polyglycerinalkoxylatester A | 0,6 |

Die Ergebnisse des Red Blood Cell-Tests aus Tabelle 6 zeigen, dass sich die erfindungsgemäßen Polyglycerinethoxylatester bei gleichzeitig guten schäumenden und sensorischen Eigenschaften positiv auf die Mildheit einer tensidischen Mischung auswirken.

Die nachfolgenden Formulierungsbeispiele zeigen exemplarische Vertreter einer Vielzahl von möglichen erfindungsgemäßen Zusammensetzungen:

Tabelle 7: weitere Formulierungsbeispiele

| | 1a | 1b | 2a | 2b | 3a | 3b | 4a | 4b | 5a | 5b | 6a | 6b | 7a | 7b |
|---|---|---|---|---|---|---|---|---|---|---|---|---|---|---|
| Wasser | ad 100% | | | | | | | | | | | | | |
| Polyglycerinalkoxylatester A | 0,5% | | 0,7% | | 1,0% | | 0,7% | | 2,0% | | 1,0% | | 0,5% | |
| Polyglycerinalkoxylatester F | | 1,0% | | 1,5% | | 1,7% | | 1,8% | | 3,0% | | 1,8% | | 1,2 |
| Sodium Laureth Sulfate | 9,0% | 9,0% | 7,0% | 7,0% | 6,0% | 6,0% | 5,0% | 5,0% | 5,0% | 5,0% | 8,0% | 8,0% | 9,0% | 9,0% |
| Sodium Lauryl Sulfate | | | | | | | | | | | | | | |
| Cocamidopropyl Betaine | 3,0% | 3,0% | 2,0% | 2,0% | 1,0% | 1,0% | 2,0% | 2,0% | | | | | | |
| Sodium Cocoamphoacetate | | | | | | | | | 2,5% | 2,5% | 2,0% | 2,0% | 3,0% | 3,0% |
| Sodium Laureth Sulfosuccinate | | | | | | | | | 2,5% | 2,5% | | | | |
| Lauryl Glucoside | | | | | | | | | | | | | | |
| Coco-Glucoside | | | | | | | 1,0% | 1,0% | | | 1,0% | 1,0% | | |
| Sodium Cocoyl Glutamate | | | | | | | | | | | | | | |
| Sodium Chloride | 0,3% | 0,3% | 0,7% | 0,7% | 1,0% | 1,0% | 1,5% | 1,5% | 1,5% | 1,5% | 1,5% | 1,5% | 1,0% | 1,0% |
| Cocamide DEA | | | 0,3% | 0,3% | | | | | 0,3% | 0,3% | 0,5% | 0,5% | 0,5% | 0,5% |
| Isostearamide MIPA; Glyceryl Laurate | 0,3% | 0,3% | | | | | 0,5% | 0,5% | | | | | | |
| Xanthan Gum | | | | | 0,1% | 0,1% | | | | | | | 0,5% | 0,5% |
| Glyceryl Glucoside | | | 0,3% | 0,3% | | | | | 0,2% | 0,2% | 0,3% | 0,3% | | |
| Sucrose Cocoate | 0,5% | 0,5% | | | | | | | | | | | 1,0% | 1,0% |
| Glycerin | 1,0% | 1,0% | | | | | | | | | 1,0% | 1,0% | 0,5% | 0,5% |
| PEG-7 Glyceryl Cocoate | | | | | 0,3% | 0,3% | 0,3% | 0,3% | 0,5% | 0,5% | | | | |
| PEG-6 Caprylic/Capric Glycerides | | | 0,2% | 0,2% | | | | | | | | | | |
| Trideceth-9 | | | | | | | | | | | 0,2% | 0,2% | | |
| Polysorbate 20 | 0,3% | 0,3% | | | | | | | | | | | 0,5% | 0,5% |
| PEG-40 Hydrogenated Castor Oil | | | 0,5% | 0,5% | | | | | 0,7% | 0,7% | | | 0,3% | 0,3% |
| Polyglyceryl-4 Caprate | | | | | 0,6% | 0,6% | | | | | | | | |
| Polyquaternium-10 | 0,2% | 0,2% | | | 0,1% | 0,1% | | | | | 0,2% | 0,2% | | |
| Hydroxypropyl Guar Hydroxy-propyltrimonium Chloride | | | 0,2% | 0,2% | | | | | 0,3% | 0,3% | | | 0,3% | 0,3% |
| Silicone Quaternium-22 | | | | | 0,2% | 0,2% | | | | | | | 0,3% | 0,3% |
| Dimethicone | | | 0,1% | 0,1% | | | | | | | 0,1% | 0,1% | | |
| Amodimethicone | | | | | 0,1% | 0,1% | | | | | 0,1% | 0,1% | | |
| Persea Gratissima Oil | | | | | 0,1% | 0,1% | | | | | | | | |
| Hydrogenated Castor Oil | | | | | | | 0,1% | 0,1% | | | 0,2% | 0,2% | | |
| Glycol Distearate | | | | | | | | | | | 0,5% | 0,5% | | |
| Zinc Pyrithione | | | | | | | | | | | 0,1% | 0,1% | | |
| Benzophenone-4 | | | 0,1% | 0,1% | | | | | | | 0,1% | 0,1% | | |
| Tetrasodium EDTA | | | | | 0,1% | 0,1% | | | | | 0,1% | 0,1% | | |
| Caffeine | 0,1% | 0,1% | | | | | | | | | 0,1% | 0,1% | 0,1% | 0,1% |
| Hydrolyzed Keratin | | | 0,1% | 0,1% | | | | | | | | | 0,1% | 0,1% |
| Panthenol | 0,1% | 0,1% | | | | | 0,1% | 0,1% | 0,1% | 0,1% | 0,1% | 0,1% | 0,1% | 0,1% |
| Citric Acid | ad pH 5,5 | | | | | | | | | | | | | |
| Perfumes, Dyes, Preservatives | q.s. | | | | | | | | | | | | | |

| | 8a | 8b | 9a | 9b | 10a | 10b | 11a | 11b | 12a | 12b | 13a | 13b |
|---|---|---|---|---|---|---|---|---|---|---|---|---|
| Wasser | ad 100% | | | | | | | | | | | |
| Polyglycerinalkoxylatester A | 0,7% | | 1,2% | | 3,5% | | 4,0% | | 3,5% | | 3,0% | |
| Polyglycerinalkoxylatester F | | 1,5% | | 2,2% | | 5,0% | | 5,0% | | | | |
| Sodium Laureth Sulfate | | | | | | | | | | | | |
| Sodium Lauryl Sulfate | 6,0% | 6,0% | 5,0% | 5,0% | | | | | 3,5% | 3,5% | 6,0% | 6,0% |
| Cocamidopropyl Betaine | 3,0% | 3,0% | 5,0% | 5,0% | 6,0% | 6,0% | | | 2,0% | 2,0% | | |
| Sodium Cocoamphoacetate | 3,0% | 3,0% | | | | | 3,0% | 3,0% | | | | |
| Sodium Laureth Sulfosuccinate | | | 2,0% | 2,0% | | | | | | | | |
| Lauryl Glucoside | | | | | 5,0% | 5,0% | 3,0% | 3,0% | | | 3,5% | 3,5% |
| Coco-Glucoside | | | | | 1,0% | 1,0% | 5,5% | 5,5% | 2,0% | 2,0% | 2,5% | 2,5% |
| Sodium Cocoyl Glutamate | | | | | 1,0% | 1,0% | 1,5% | 1,5% | 0,5% | 0,5% | | |
| Sodium Chloride | 1,5% | 1,5% | 1,0% | 1,0% | | | | | | | 1,0% | 1,0% |
| Cocamide DEA | 1,0% | 1,0% | | | 1,0% | 1,0% | | | | | | |
| Isostearamide MIPA; Glyceryl Laurate | | | | | | | 0,2% | 0,2% | 1,0% | 1,0% | 0,5% | 0,5% |
| Xanthan Gum | | | | | 0,7% | 0,7% | 2,0% | 2,0% | - | - | - | - |
| Glyceryl Glucoside | 0,5% | 0,5% | 0,3% | 0,3% | | | 0,2% | 0,2% | | | | |
| Sucrose Cocoate | | | 0,3% | 0,3% | 0,2% | 0,2% | - | - | 1,0% | 1,0% | 1,0% | 1,0% |
| Glycerin | 1,0% | 1,0% | 0,3% | 0,3% | 0,4% | 0,4% | 1,5% | 1,5% | 0,5% | 0,5% | 1,0% | 1,0% |
| PEG-7 Glyceryl Cocoate | | | | | | | | | | | 0,5% | 0,5% |
| PEG-6 Caprylic/Capric Glycerides | | | 0,3% | 0,3% | | | | | 0,2% | 0,2% | 0,2% | 0,2% |
| Trideceth-9 | | | 0,2% | 0,2% | | | | | | | | |
| Polysorbate 20 | | | | | | | | | 0,3% | 0,3% | 0,2% | 0,2% |
| PEG-40 Hydrogenated Castor Oil | | | 0,5% | 0,5% | | | | | 1,0% | 1,0% | - | - |
| Polyglyceryl-4 Caprate | 2,0% | 2,0% | | | 0,5% | 0,5% | | | | | 0,5% | 0,5% |
| Polyquaternium-10 | 0,1% | 0,1% | | | | | | | 0,2% | 0,2% | | |
| Hydroxypropyl Guar Hydroxy-propyltrimonium Chloride | 0,2% | 0,2% | 0,2% | 0,2% | 0,2% | 0,2% | 0,2% | 0,2% | | | | |
| Silicone Quaternium-22 | | | 0,3% | 0,3% | | | | | | | | |
| Dimethicone | | | | | | | | | 0,1% | 0,1% | | |
| Amodimethicone | 0,1% | 0,1% | 0,1% | 0,1% | | | | | 0,5% | 0,5% | | |
| Persea Gratissima Oil | 0,1% | 0,1% | | | 0,1% | 0,1% | 0,2% | 0,2% | | | | |
| Hydrogenated Castor Oil | | | | | | | | | 0,1% | 0,1% | 0,2% | 0,2% |
| Glycol Distearate | | | 0,5% | 0,5% | | | 0,3% | 0,3% | 0,5% | 0,5% | 0,5% | 0,5% |
| Zinc Pyrithione | | | - | - | - | - | - | - | 0,1% | 0,1% | | |
| Benzophenone-4 | 0,1% | 0,1% | 0,1% | 0,1% | - | - | 0,1% | 0,1% | 0,1% | 0,1% | | |
| Tetrasodium EDTA | | | 0,1% | 0,1% | - | - | - | - | 0,1% | 0,1% | | |
| Caffeine | | | | | - | - | - | - | 0,1% | 0,1% | | |
| Hydrolyzed Keratin | | | | | 0,1% | 0,1% | 0,2% | 0,2% | 0,1% | 0,1% | | |
| Panthenol | 0,1% | 0,1% | 0,1% | 0,1% | 0,1% | 0,1% | | | 0,1% | 0,1% | 0,1% | 0,1% |
| Citric Acid | ad pH 5,5 | | | | | | | | | | | |
| Perfumes, Dyes, Preservatives | q.s. | | | | | | | | | | | |

Tabelle 8: weitere Formulierungsbeispiele

| | 14a | 14b | 15a | 15b | 16a | 16b | 17a | 17b | 18a | 18b | 19a | 19b | 20a | 20b |
|---|---|---|---|---|---|---|---|---|---|---|---|---|---|---|
| Wasser | ad 100% | | | | | | | | | | | | | |
| Polyglycerinalkoxylatester A | 0,3% | | 0,5 | | 0,6 | | 1,5% | | 0,5% | | 0,4 | | 1,3% | |
| Polyglycerinalkoxylatester F | | 0,7 | | 1,5% | | 1,7% | | 2,5% | | 1,4% | | 1,0% | | 2,5% |
| Sodium Laureth Sulfate | 9% | 9% | 8,0% | 8,0% | 6,0% | 6,0% | | | 6% | 6% | 4,5% | 4,5% | | |
| Coco-Betaine | 3% | 3% | 3,0% | 3,0% | | | 5,5% | 5,5% | | | | | | |
| Cocamidopropyl Betaine | | | | | 2,0% | 2,0% | | | 1,5% | 1,5% | | | 5,0% | 5,0% |
| Sodium Cocoamphoacetate | | | | | 2,0% | 2,0% | 3,0% | 3,0% | | | 4,5% | 4,5% | | |
| Disodium Lauryl Sulfosuccinate | | | | | 1,0% | 1,0% | | | 1,5% | 1,5% | | | | |
| Decyl Glucoside | | | 1,0% | 1,0% | | | 2,0% | 2,0% | 1,0% | 1,0% | | | 3,0% | 3,0% |
| Sodium Cocoyl Glutamate | | | | | | | 2,5% | 2,5% | | | | | | |
| Sodium Cocoyl Glycinate | | | | | | | | | | | | | 2,0% | 2,0% |
| Sodium Lauroyl Isethionate | | | | | | | | | | | | | | |
| Sodium Chloride | 0,7% | 0,7% | 0,7% | 0,7% | 1,0% | 1,0% | | | 1,0% | 1,0% | 1,7% | 1,7% | 1,5% | 1,5% |
| Cocamide MEA | | | 0,5% | 0,5% | | | 1,0% | 1,0% | | | | | | |
| Xanthan Gum | | | | | | | | | | | | | | |
| Hydroxyethyl Ethylcellulose | | | | | | | 0,3% | 0,3% | | | | | | |
| Acrylates / C10-30 Alkyl Acrylate Crosspolymer | 0,5% | 0,5% | | | | | | | | | | | 0,5% | 0,5% |
| Stearic Acid | | | | | 0,3% | 0,3% | | | | | | | | |
| Sucrose Cocoate | 0,5% | 0,5% | 0,4% | 0,4% | | | 1,0% | 1,0% | | | 0,3% | 0,3% | | |
| Glycerin | 1,5% | 1,5% | 0,3% | 0,3% | | | 0,5% | 0,5% | 1,0% | 1,0% | | | 0,3% | 0,3% |
| PEG-40 Hydrogenated Castor Oil | | | 1,0% | 1,0% | | | | | | | | | | |
| Polyglyceryl-4 Caprate | 0,5% | 0,5% | | | | | 0,5% | 0,5% | | | | | | |
| Polyquaternium-11 | | | 0,2% | 0,2% | | | | | | | | | 0,1% | 0,1% |
| Guar Hydroxypropyltrimonium Chloride | | | | | 0,3% | 0,3% | 0,2% | 0,2% | | | | | 0,2% | 0,2% |
| Dimethicone | | | 0,3% | 0,3% | | | | | | | | | | |
| Aminopropyl Dimethicone | | | | | 0,5% | 0,5% | | | | | | | | |
| PEG-3 Distearate | | | 0,5% | 0,5% | | | | | | | | | | |
| Benzophenone-4 | | | 0,1% | 0,1% | 0,2% | 0,2% | | | | | | | | |
| Menthol | 0,1% | 0,1% | | | 0,1% | 0,1% | | | 0,1% | 0,1% | | | | |
| Caffeine | | | | | 0,1% | 0,1% | | | | | | | 0,1% | 0,1% |
| Benzyl Alcohol | 0,1% | 0,1% | | | | | | | | | | | | |
| Coumarin | 0,1% | 0,1% | | | 0,1% | 0,1% | 0,1% | 0,1% | | | | | | |
| Hydrolyzed Wheat Protein | | | | | 0,1% | 0,1% | | | 0,1% | 0,1% | | | 0,1% | 0,1% |
| Panthenol | 0,1% | 0,1% | 0,1% | 0,1% | | | 0,1% | 0,1% | 0,1% | 0,1% | 0,1% | 0,1% | 0,1% | 0,1% |
| Sodium Hydroxide, 25% | | | | | 0,6% | 0,6% | | | | | | | 0,8% | 0,8% |
| Citric Acid | ad pH 6,0 | | | | | | | | | | | | | |
| Perfumes, Dyes, Preservatives | q.s | | | | | | | | | | | | | |

| | 21a | 21b | 22a | 22b | 23a | 23b | 24a | 24b | 25a | 25b | 26a | 26b |
|---|---|---|---|---|---|---|---|---|---|---|---|---|
| Wasser | | | | | | | | | | | | |
| Polyglycerinalkoxylatester A | 3,5% | | 2,7% | | 1,0% | | 1,2% | | 2,5% | | | |
| Polyglycerinalkoxylatester F | | 5,0% | | 4,5% | | 2,2% | 2,5% | 2,5% | | 3,5% | | |
| Sodium Laureth Sulfate | | | | | 4,0% | 4,0% | | | | | | |
| Coco-Betaine | | | | | 2,0% | 2,0% | | | | | | |
| Cocamidopropyl Betaine | | | 3,5% | 3,5% | | | 5,0% | 5,0% | 7,0% | 7,0% | 6,0% | 6,0% |
| Sodium Cocoamphoacetate | 5,0% | 5,0% | | | 2,0% | 2,0% | | | | | 2,0% | 2,0% |
| Disodium Lauryl Sulfosuccinate | | | | | 1,0% | 1,0% | | | | | 2,0% | 2,0% |
| Decyl Glucoside | 4,0% | 4,0% | 5,0% | 5,0% | | | 2,0% | 2,0% | | | | |
| Sodium Cocoyl Glutamate | 2,0% | 2,0% | 2,5% | 2,5% | 1,5% | 1,5% | 1,0% | 1,0% | | | | |
| Sodium Cocoyl Glycinate | | | | | | | | | 3,0% | 3,0% | | |
| Sodium Lauroyl Isethionate | 1,0% | 1,0% | | | 1,0% | 1,0% | 0,5% | 0,5% | 2,0% | 2,0% | | |
| Sodium Chloride | | | | | 1,0% | 1,0% | 1,0% | 1,0% | 1,0% | 1,0% | 1,0% | 1,0% |
| Cocamide MEA | 0,2% | 0,2% | 0,5% | 0,5% | | | 0,3% | 0,3% | | | | |
| Xanthan Gum | | | 0,1% | 0,1% | 0,2% | 0,2% | 0,3% | 0,3% | 0,2% | 0,2% | | |
| Hydroxyethyl Ethylcellulose | 0,1% | 0,1% | | | | | | | 0,5% | 0,5% | | |
| Acrylates / C10-30 Alkyl Acrylate Crosspolymer | 0,4% | 0,4% | | | | | | | | | | |
| Stearic Acid | | | | | 0,1% | 0,1% | 0,5% | 0,5% | 0,5% | 0,5% | | |
| Sucrose Cocoate | | | 0,2% | 0,2% | 0,3% | 0,3% | 0,3% | 0,3% | | | 0,2% | 0,2% |
| Glycerin | 0,5% | 0,5% | 1,0% | 1,0% | | | 1,0% | 1,0% | 2,0% | 2,0% | 1,5% | 1,5% |
| PEG-40 Hydrogenated Castor Oil | | | | | 0,3% | 0,3% | | | | | | |
| Polyglyceryl-4 Caprate | 2,6% | 2,6% | | | | | | | | | | |
| Polyquaternium-11 | | | | | 0,2% | 0,2% | 0,3% | 0,3% | | | 0,1% | 0,1% |
| Guar Hydroxypropyltrimonium Chloride | 0,3% | 0,3% | 0,2% | 0,2% | 0,1% | 0,1% | | | | | | |
| Dimethicone | | | | | 0,2% | 0,2% | | | | | | |
| Aminopropyl Dimethicone | | | | | | | | | | | | |
| PEG-3 Distearate | | | | | 0,5% | 0,5% | | | 0,5% | 0,5% | | |
| Benzophenone-4 | | | | | 0,2% | 0,2% | 0,1% | 0,1% | | | | |
| Menthol | 0,1% | 0,1% | | | 0,1% | 0,1% | 0,1% | 0,1% | | | | |
| Caffeine | | | | | 0,1% | 0,1% | 0,1% | 0,1% | | | 0,1% | 0,1% |
| Benzyl Alcohol | 0,1% | 0,1% | | | 0,1% | 0,1% | | | 0,1% | 0,1% | | |
| Coumarin | | | 0,1% | 0,1% | | | 0,1% | 0,1% | | | | |
| Hydrolyzed Wheat Protein | | | 0,2% | 0,2% | 0,1% | 0,1% | | | | | | |
| Panthenol | 0,1% | 0,1% | 0,1% | 0,1% | | | 0,1% | 0,1% | 0,1% | 0,1% | | |
| Sodium Hydroxide, 25% | 0,5% | 0,5% | | | | | | | | | | |
| Citric Acid | ad pH 6,0 | | | | | | | | | | | |
| Perfumes, Dyes, Preservatives | q.s. | | | | | | | | | | | |

**Patentansprüche**

1. Polyglycerinalkoxylatester der allgemeinen Formel (I)

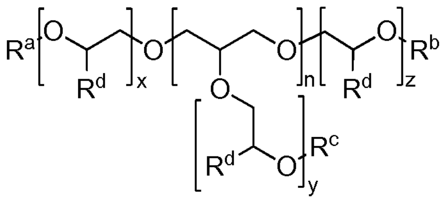

allgemeine Formel (I)

mit

$R^a$, $R^b$, $R^c$ = unabhängig voneinander, gleich oder verschieden, ausgewählt aus H oder Acylrest einer organischen Säure, bevorzugt H oder Acylrest einer Fettsäure, besonders bevorzugt H oder Acylrest einer Fettsäure mit 16 bis 22 Kohlenstoffatomen,
mit der Maßgabe, dass pro Molekül im Mittel 3 bis 6, besonders bevorzugt 3,5 bis 5,5, insbesondere bevorzugt 4 bis 5 der Reste $R^a$, $R^b$, $R^c$ ungleich H,
$R^d$ = unabhängig voneinander, gleich oder verschieden, ausgewählt aus H, Alkyl- oder Aryl-, bevorzugt H, Methyl-, Ethyl-, besonders bevorzugt H oder Methyl, insbesondere H,
n = 1,5 bis 16, bevorzugt 2 bis 14, besonders bevorzugt 3 bis 11, ganz besonders bevorzugt 4 bis 9,
x, y, z = unabhängig voneinander, gleich oder verschieden, 0 bis 200, bevorzugt 30 bis 100, besonders bevorzugt 40 bis 80,
mit der Maßgabe, dass die Gesamtsumme $x + n \cdot y + z$ pro Molekül im Mittel 251 bis 750, bevorzugt 300 bis 600, besonders bevorzugt 350 bis 550, beträgt.

**2.** Polyglycerinalkoxylatester nach Anspruch 1, **dadurch gekennzeichnet, dass** $R^a$
, $R^b$, $R^c$ = H oder Acylrest einer Fettsäure mit 16 bis 22 Kohlenstoffatomen,
mit der Maßgabe, dass pro Molekül im Mittel 3,5 bis 6 der Reste $R^a$, $R^b$, $R^c$ ungleich H,
$R^d$ = H,
n = 3 bis 11,
x, y, z = unabhängig voneinander, gleich oder verschieden, 30 bis 200,
mit der Maßgabe, dass die Gesamtsumme $x + n \cdot y + z$ pro Molekül im Mittel 300 bis 750 beträgt.

**3.** Verfahren zur Herstellung von Polyglycerinalkoxylatestern umfassend die Verfahrensschritte

A) Bereitstellen eine Polyglycerins mit einem Polymerisationsgrad n von 1,5 bis 16, bevorzugt 2 bis 14, besonders bevorzugt 3 bis 11, ganz besonders bevorzugt 4 bis 9,
B) Alkoxylierung mit 251 bis 750, bevorzugt 300 bis 600, besonders bevorzugt 350 bis 550, Mol Alkylenoxid ausgewählt aus der Gruppe Ethylenoxid, Propylenoxid, Butylenoxid und Dodecenoxid, insbesondere Ethylenoxid, pro im gesamten Verfahren eingesetzten Mol Polyglycerin, und
C) Veresterung mit 3 bis 6, besonders bevorzugt 3,5 bis 5,5, insbesondere bevorzugt 4 bis 5, Mol mindestens einer ausgewählt aus organischen Säuren, bevorzugt Fettsäuren, insbesondere Fettsäuren mit 16 bis 22 Kohlenstoffatomen, pro im gesamten Verfahren eingesetzten Mol Polyglycerin.

**4.** Verfahren nach Anspruch 3, **dadurch gekennzeichnet, dass** die Verfahrensschritte in der Reihenfolge "A), C), B)" durchgeführt werden.

**5.** Verfahren nach Anspruch 3 oder 4, **dadurch gekennzeichnet, dass** in Verfahrensschritt A) ein Polyglycerin mit einem Polymerisationsgrad von 3 bis 11,
in Verfahrensschritt B) 300 bis 750 Mol Ethylenoxid, pro im gesamten Verfahren eingesetzten Mol Polyglycerin, und in Verfahrensschritt C) 3,5 bis 6 Mol mindestens einer ausgewählt aus Fettsäuren mit 16 bis 22 Kohlenstoffatomen pro im gesamten Verfahren eingesetzten Mol Polyglycerin,
eingesetzt werden.

**6.** Polyglycerinalkoxylatester erhältlich nach einem Verfahren nach mindestens einem der Ansprüche 3 bis 5.

**7.** Formulierungen enthaltend mindestens ein Polyglycerinalkoxylatester nach mindestens einem der Ansprüche 1 bis 3 oder 6.

**8.** Wässrige Formulierung nach Anspruch 7 enthaltend mindestens ein Tensid.

9. Formulierung nach Anspruch 8 enthaltend ein oder mehrere Tenside in einer Gesamtmenge von 0,1 Gew.-% bis 20 Gew.-%, bevorzugt 1,0 Gew.-% bis 15 Gew.-%, insbesondere 5,0 Gew.-% bis 10 Gew.-%, wobei sich die Gewichtsprozente auf die Gesamtzusammensetzung beziehen.

10. Wässrige Formulierung nach Anspruch 7 enthaltend mindestens einen gegebenenfalls alkoxylierten Alkohol und/oder mindestens einen gegebenenfalls alkoxylierten Carbonsäureester.

11. Formulierung nach Anspruch 10, **dadurch gekennzeichnet, dass** der Polyglycerinalkoxylatester in einer Menge von 30 bis 70 Gew.-% bezogen auf die Gesamtformulierung enthalten ist.

12. Verwendung mindestens eines Polyglycerinalkoxylatesters nach mindestens einem der Ansprüche 1 bis 3 oder 6 als Verdicker einer Formulierung, insbesondere einer Formulierung nach einem der Ansprüche 7 bis 9.

13. Verwendung mindestens eines Polyglycerinalkoxylatesters nach mindestens einem der Ansprüche 1 bis 3 oder 6 oder einer Formulierung nach einem der Ansprüche 7 bis 9 zur Hautpflege.

14. Verwendung mindestens eines Polyglycerinalkoxylatesters nach mindestens einem der Ansprüche 1 bis 3 oder 6 zur Schaumstabilisierung in einer Formulierung, insbesondere einer Formulierung nach einem der Ansprüche 7 bis 9.

15. Verwendung mindestens eines Polyglycerinalkoxylatesters nach mindestens einem der Ansprüche 1 bis 3 oder 6 zur Reduktion der Reizwirkung von kosmetischen Formulierungen auf insbesondere Haut und/oder Augen.

## Claims

1. Polyglycerol alkoxylate esters of the general formula (I)

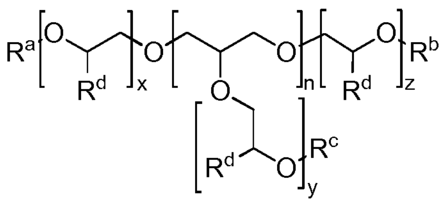

general formula (I)

with

$R^a$, $R^b$, $R^c$ = identical or different and independently selected from H and acyl radical of an organic acid, preferably H and acyl radical of a fatty acid, more preferably H and acyl radical of a fatty acid having 16 to 22 carbon atoms, with the proviso that an average of 3 to 6, more preferably 3.5 to 5.5 and especially preferably 4 to 5 of the $R^a$, $R^b$, $R^c$ radicals per molecule are not H,
$R^d$ = identical or different and independently selected from H, alkyl- and aryl-, preferably H, methyl-, ethyl-, more preferably H and methyl, especially H,
n = 1.5 to 16, preferably 2 to 14, more preferably 3 to 11, most preferably 4 to 9,
x, y, z = identical or different and independently 0 to 200, preferably 30 to 100, more preferably 40 to 80,
with the proviso that the sum total of $x + n \cdot y + z$ for each molecule averages 251 to 750, preferably 300 to 600, more preferably 350 to 550.

2. Polyglycerol alkoxylate esters according to Claim 1,
**characterized in that**
$R^a$, $R^b$, $R^c$ = H or acyl radical of a fatty acid having 16 to 22 carbon atoms,
with the proviso that an average of 3.5 to 6 of the $R^a$, $R^b$, $R^c$ radicals per molecule are not H,
$R^d$ = H,
n = 3 to 11,
x, y, z = identical or different and independently 30 to 200,
with the proviso that the sum total of $x + n \cdot y + z$ for each molecule averages 300 to 750.

**3.** Process for preparing polyglycerol alkoxylate esters, comprising the process steps of

A) providing a polyglycerol having a polymerization level n of 1.5 to 16, preferably 2 to 14, more preferably 3 to 11, most preferably 4 to 9,
B) alkoxylating with 251 to 750, preferably 300 to 600 and more preferably 350 to 550 mol of alkylene oxide selected from the group of ethylene oxide, propylene oxide, butylene oxide and dodecene oxide, especially ethylene oxide, per mole of polyglycerol used in the overall process, and
C) esterifying with 3 to 6, more preferably 3.5 to 5.5 and especially preferably 4 to 5 mol of at least one selected from organic acids, preferably fatty acids, especially fatty acids having 16 to 22 carbon atoms, per mole of polyglycerol used in the overall process.

**4.** Process according to Claim 3, **characterized in that** the process steps are conducted in the sequence "A), C), B)".

**5.** Process according to Claim 3 or 4, **characterized in that**
a polyglycerol having a polymerization level of 3 to 11 is used in process step A),
300 to 750 mol of ethylene oxide per mole of polyglycerol used in the overall process is used in process step B) and 3.5 to 6 mol of at least one selected from fatty acids having 16 to 22 carbon atoms per mole of polyglycerol used in the overall process is used in process step C).

**6.** Polyglycerol alkoxylate ester obtainable by a process according to at least one of Claims 3 to 5.

**7.** Formulations comprising at least one polyglycerol alkoxylate ester according to at least one of Claims 1 to 3 or 6.

**8.** Aqueous formulation according to Claim 7 comprising at least one surfactant.

**9.** Formulation according to Claim 8, comprising one or more surfactants in a total amount of 0.1% by weight to 20% by weight, preferably 1.0% by weight to 15% by weight, especially 5.0% by weight to 10% by weight, where the percentages by weight relate to the overall composition.

**10.** Aqueous formulation according to Claim 7, comprising at least one optionally alkoxylated alcohol and/or at least one optionally alkoxylated carboxylic ester.

**11.** Formulation according to Claim 10, **characterized in that** the polyglycerol alkoxylate ester is present in an amount of 30% to 70% by weight, based on the overall formulation.

**12.** Use of at least one polyglycerol alkoxylate ester according to at least one of Claims 1 to 3 or 6 as thickener for a formulation, especially a formulation according to any of Claims 7 to 9.

**13.** Use of at least one polyglycerol alkoxylate ester according to at least one of Claims 1 to 3 or 6 or of a formulation according to any of Claims 7 to 9 for skincare.

**14.** Use of at least one polyglycerol alkoxylate ester according to at least one of Claims 1 to 3 or 6 for foam stabilization in a formulation, especially a formulation according to any of Claims 7 to 9.

**15.** Use of at least one polyglycerol alkoxylate ester according to at least one of Claims 1 to 3 or 6 for reducing irritation by cosmetic formulations, especially on skin and/or eyes.

## Revendications

**1.** Ester de type alcoxylate de polyglycérol de formule générale (I)

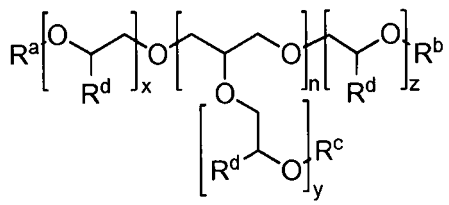

Formule générale (I)

où

$R^a$, $R^b$, $R^c$ = indépendamment les uns des autres, identiques ou différents, choisis parmi H ou le radical acyle d'un acide organique, de préférence H ou le radical acyle d'un acide gras, de manière particulièrement préférée H ou un radical acyle d'un acide gras comprenant 16 à 22 atomes de carbone, à condition que par molécule, en moyenne 3 à 6, de manière particulièrement préférée 3,5 à 5,5, en particulier de préférence 4 à 5 des radicaux $R^a$, $R^b$, $R^c$ soient différents de H,

$R^d$ = indépendamment les uns des autres, identiques ou différents, choisis parmi H, alkyle ou aryle, de préférence H, méthyle, éthyle, de manière particulièrement préférée H ou méthyle, en particulier H,

n = 1,5 à 16, de préférence 2 à 14, de manière particulièrement préférée 3 à 11, de manière tout particulièrement préférée 4 à 9,

x, y, z = indépendamment les uns des autres, identiques ou différents, 0 à 200, de préférence 30 à 100, de manière particulièrement préférée 40 à 80, à condition que la somme totale $x+n \cdot y+z$ par molécule vaille en moyenne 251 à 750, de préférence 300 à 600, de manière particulièrement préférée 350 à 550.

**2.** Ester de type alcoxylate de polyglycérol selon la revendication 1, **caractérisé en ce que**
$R^a$, $R^b$, $R^c$ = H ou le radical acyle d'un acide gras comprenant 16 à 22 atomes de carbone, à condition que par molécule, en moyenne 3,5 à 6 des radicaux $R^a$, $R^b$, $R^c$ soient différents de H,
$R^d$ = H,
n = 3 à 11,
x, y, z = indépendamment les uns des autres, identiques ou différents, 30 à 200, à condition que la somme totale $x+n \cdot y+z$ par molécule vaille en moyenne 300 à 750.

**3.** Procédé pour la préparation d'esters de type alcoxylate de polyglycérol, comprenant les étapes de procédé

A) préparation d'un polyglycérol présentant un degré de polymérisation n de 1,5 à 16, de préférence de 2 à 14, de manière particulièrement préférée de 3 à 11, de manière tout particulièrement préférée de 4 à 9,

B) alcoxylation avec 251 à 750, de préférence 300 à 600, de manière particulièrement préférée 350 à 550, moles d'oxyde d'alkylène choisi dans le groupe oxyde d'éthylène, oxyde de propylène, oxyde de butylène et oxyde de dodécène, en particulier oxyde d'éthylène, par mole de polyglycérol utilisée dans l'ensemble du procédé et

C) estérification par 3 à 6, de manière particulièrement préférée 3,5 à 5,5, en particulier de préférence 4 à 5, moles d'au moins un acide choisi parmi les acides organiques, de préférence les acides gras, en particulier les acides gras comprenant 16 à 22 atomes de carbone, par mole de polyglycérol utilisée dans l'ensemble du procédé.

**4.** Procédé selon la revendication 3, **caractérisé en ce que** les étapes de procédé sont réalisées dans l'ordre "A), C), B)".

**5.** Procédé selon la revendication 3 ou 4, **caractérisé en ce qu'**on utilise
dans l'étape de procédé A), un polyglycérol présentant un degré de polymérisation de 3 à 11,
dans l'étape de procédé B), 300 à 750 moles d'oxyde d'éthylène par mole de polyglycérol utilisée dans l'ensemble du procédé,
dans l'étape de procédé C), 3,5 à 6 moles d'au moins un acide choisi parmi les acides gras comprenant 16 à 22 atomes de carbone par mole de polyglycérol utilisée dans l'ensemble du procédé.

**6.** Ester de type alcoxylate de polyglycérol pouvant être obtenu selon un procédé selon l'une quelconque des revendications 3 à 5.

7. Formulations contenant au moins un ester de type alcoxylate de polyglycérol selon au moins l'une quelconque des revendications 1 à 3 ou 6.

8. Formulation aqueuse selon la revendication 7 contenant au moins un tensioactif.

9. Formulation selon la revendication 8, contenant un ou plusieurs tensioactifs en une quantité totale de 0,1% en poids à 20% en poids, de préférence de 1,0% en poids à 15% en poids, en particulier de 5,0% en poids à 10% en poids, les % en poids se rapportant à la composition totale.

10. Formulation aqueuse selon la revendication 7 contenant au moins un alcool le cas échéant alcoxylé et/ou au moins un ester d'acide carboxylique le cas échéant alcoxylé.

11. Formulation selon la revendication 10, **caractérisée en ce que** l'ester de type alcoxylate de polyglycérol est contenu en une quantité de 30 à 70% en poids par rapport à la formulation totale.

12. Utilisation d'au moins un ester de type alcoxylate de polyglycérol selon au moins l'une quelconque des revendications 1 à 3 ou 6 comme épaississant d'une formulation, en particulier d'une formulation selon l'une quelconque des revendications 7 à 9.

13. Utilisation d'au moins un ester de type alcoxylate de polyglycérol selon au moins l'une quelconque des revendications 1 à 3 ou 6 ou d'une formulation selon l'une quelconque des revendications 7 à 9 pour les soins de la peau.

14. Utilisation d'au moins un ester de type alcoxylate de polyglycérol selon au moins l'une quelconque des revendications 1 à 3 ou 6 pour la stabilisation de la mousse dans une formulation, en particulier une formulation selon l'une quelconque des revendications 7 à 9.

15. Utilisation d'au moins un ester de type alcoxylate de polyglycérol selon au moins l'une quelconque des revendications 1 à 3 ou 6 pour la réduction de l'effet irritant de formulations cosmétiques en particulier sur la peau et/ou les yeux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0809527 A
- EP 0264826 A
- DE 10124547
- WO 0108481 A
- DE 3239564
- DE 19753108
- DE 2024051
- EP 1344518 A
- EP 1518900 A


### In der Beschreibung aufgeführte Nicht-Patentliteratur


- **CASSEL et al.** Original synthesis of linear, branched and cyclic oligoglycerol standards. *J. Org. Chem.,* 2001, 875-896
- Alkylene_Oxides_and_Their_Polymers. **F. E. BAILEY ; J. V. KOLESKE.** Ullmann's Encyclopedia of Industrial Chemistry. Marcel Dekker Inc, 1991
- **FETTE ; SEIFEN.** *Anstrichm,* 1980, vol. 82, 93
- **TENSIDE.** *Deterg,* 1986, vol. 23, 320